# EUROPEAN PATENT APPLICATION

(11) **EP 4 790 065 A1**
(43) Date of publication of application: **12.08.2026**
(21) Application number: 24874729.7
(22) Date of filing: 04.10.2024
(51) Int. Cl.: C07K 14/165, A61K 39/215, A61P 31/14, C12N 15/50, C12N 15/63

(54) **CORONAVIRUS SPIKE PROTEIN VARIANTS**

(30) Priority: 06.10.2023 JP 2023174297
(71) Applicant: Shionogi & Co., Ltd, Osaka-shi, Osaka 541-0045 (JP); KOTAI Biotechnologies, Inc., Minoh-shi, Osaka 562-0035 (JP)
(72) Inventor: NAKAGAWA, Takayuki, Osaka-shi, Osaka 541-0045 (JP); HAYASHIDA, Marina, Osaka-shi, Osaka 541-0045 (JP); SATO, Masaaki, Osaka-shi, Osaka 541-0045 (JP); AKIYAMA, Miho, Osaka-shi, Osaka 541-0045 (JP); OTA, Takeshi, Osaka-shi, Osaka 541-0045 (JP); SAKO, Yusuke, Osaka-shi, Osaka 541-0045 (JP); KITAHATA, Shun, Osaka-shi, Osaka 541-0045 (JP); FUTAMATA, Ryota, Osaka-shi, Osaka 541-0045 (JP); YAMASHITA, Kazuo, Minoh-shi, Osaka 562-0035 (JP); WANG, Po-Hung, Minoh-shi, Osaka 562-0035 (JP)
(74) Representative: Vossius & Partner Patentanwälte Rechtsanwälte mbB
(86) International application number: PCT/JP2024/035568
(87) International publication number: WO 2025/075129

(57) **Abstract**

Coronavirus spike protein variants for use in antigens for vaccines for coronavirus infection are provided.

A coronavirus spike protein variant comprising:
(a) a receptor binding domain (RBD) consisting of an amino acid sequence set forth in SEQ ID NO: 1;
(b) an RBD consisting of an amino acid sequence obtained by substituting, deleting, inserting, or adding one to several amino acids in the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1; or
(c) an RBD consisting of an amino acid sequence that has at least 90% sequence identity to the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1.

## Description

### [Technical Field]

The present invention relates to coronavirus spike protein variants (also referred to as "the (coronavirus) spike protein variants of the present invention") that are suitable for antigens of novel coronavirus vaccines, or an immunogenic composition containing the spike protein variants (also referred to as an "immunogenic composition of the present invention").

### [Background Art]

SARS-CoV-1 (Severe acute respiratory syndrome-related coronavirus, also referred to as "SARS-1") is an RNA virus belonging to the order Nidovirales and the family Coronaviridae, and causes severe acute respiratory syndrome (SARS). SARS first emerged in November 2002 and caused outbreaks of SARS from 2002 to 2004. SARS-CoV-2 is also an RNA virus belonging to the order Nidovirales and the family Coronaviridae, and causes acute respiratory symptoms. The infectious disease caused by SARS-CoV-2 (COVID-19) has spread worldwide since its emergence in 2019, resulting in a global pandemic. The infectious disease caused by SARS-CoV-2 is referred to as COVID-19.

BNT162b2 (BioNTech and Pfizer) and mRNA-1273 (Moderna) are mRNA vaccines for the prevention of COVID-19, and have been demonstrated to exhibit high efficacy in preventing COVID-19 (Non Patent Documents 1 and 2).

The above-mentioned initial mRNA vaccines each encoded the spike protein of the ancestral strain; however, with the emergence of the Omicron strain exhibiting high immune evasion capability, bivalent vaccines in which the spike protein of the Omicron strain was added were developed to address the Omicron strain (Non Patent Document 3).

Coronavirus variants that evade immunity induced by coronavirus vaccines and coronavirus infection have continued to be reported (Non Patent Document 4).

In addition, SARS-CoV-1 and SARS-CoV-2 are considered to be zoonotic infectious diseases of animal origin, and coronaviruses closely related to SARS-CoV-2 have been isolated from bats and other animals (Non Patent Document 5). There is concern that future pandemics caused by novel coronaviruses may occur.

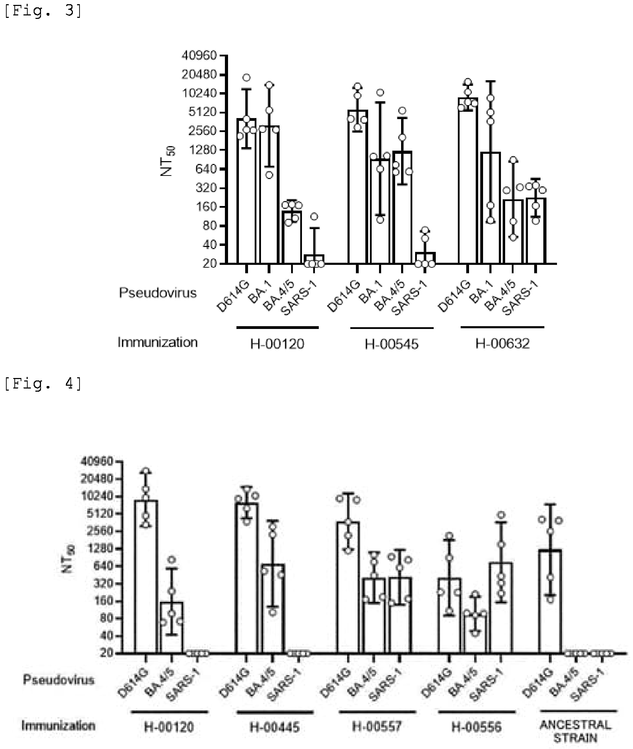

Non Patent Document 6 demonstrates that mRNA vaccines encoding various chimeric spike proteins, in which the RBD, NTD, and S2 are derived from the sequences of SARS-CoV-1, SARS-CoV-2, or the like, exhibit broad cross-reactivity. Non Patent Document 7 discloses a spike protein in which the RBD of the Delta strain is inserted at the N-terminal side of the RBD of the spike protein of the Omicron strain (BA.1 strain), directed toward vaccine antigens exhibiting broad cross-reactivity against various variants of concern (VOC). Non Patent Document 8 is a review on universal vaccines against SARS-CoV-2 variants, and various designs of spike proteins are disclosed in Fig. 3 of the document. Non Patent Document 9 discloses nanoparticles carrying the RBDs of eight sarbecoviruses, including SARS-CoV-2 and WIV-1, designed to elicit broad reactivity. However, this approach does not induce antibodies against epitopes in variable sequences of each RBD, and instead aims to elicit antibodies against conserved regions, thereby achieving broad reactivity through antibody induction against conserved regions. Non Patent Document 10 discloses three antigens in which the receptor-binding motif (RBM) of the RBD is derived from SARS-CoV-2, while the remaining portion outside the RBM is derived from SARS-CoV-1, WIV-1, SARS-CoV-2, or other strains.

However, none of the above documents describes the coronavirus spike protein variants according to the present invention.

### [Prior Art Documents]

### [Non Patent Documents]

[Non Patent Document 1] N Engl J Med. 2021 Nov 4; 385(19): 1761-1773.
[Non Patent Document 2] N Engl J Med. 2021 Feb 4; 384(5): 403-416.
[Non Patent Document 3] Lancet Infect Dis. 2023 Aug 2; S1473-3099(23) 00373-0.
[Non Patent Document 4] Nat Rev Microbiol. 2023 Mar; 21(3): 162-177.
[Non Patent Document 5] Cell. 2021 Aug 19; 184 (17): 4380-4391.
[Non Patent Document 6] Science. 2021 Aug 27; 373(6558): 991-998.
[Non Patent Document 7] NPJ Vaccines. 2022 Dec 19; 7(1): 167.
[Non Patent Document 8] Mol Ther Nucleic Acids. 2022 Dec 13; 30: 465-476.
[Non Patent Document 9] Science. 2022 Aug 5; 377 (6606): eabq0839.
[Non Patent Document 10] Cell Rep. 2022 Mar 22; 38(12): 110561.

### [SUMMARY OF THE INVENTION]

### [TECHNICAL PROBLEMS TO BE SOLVED BY THE INVENTION]

An object of the present invention is to provide an antigen or the like that has immunogenicity against betacoronavirus including sarbecovirus and that is suitable for a vaccine for prevention of coronavirus infection including COVID-19.

### [MEANS FOR SOLVING THE PROBLEM]

The inventors of the present invention completed the present invention as a result of repeated studies in order to solve the above-mentioned problems.

The present invention relates to the following Items (1) to (25).
(1) A coronavirus spike protein variant comprising:
(a) a receptor binding domain (RBD) consisting of an amino acid sequence set forth in SEQ ID NO: 1;
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1; or
(c) an RBD consisting of an amino acid sequence that has at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1.

(1-1) The coronavirus spike protein variant according to Item 1, comprising (b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 18, 20, 26, 29, 44, 45, 56, 65, 74, 75, and 78 in the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1, or
(c) an RBD consisting of an amino acid sequence obtained by substituting, deleting, inserting, or adding amino acids at positions other than positions 18, 20, 26, 29, 44, 45, 56, 65, 74, 75, and 78 in the amino acid sequence set forth in SEQ ID NO: 1, having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1.
(1-2) A coronavirus spike protein variant comprising (a) an RBD consisting of an amino acid sequence set forth in SEQ ID NO: 32,
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32, or
(c) an RBD consisting of an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32.

(1-3) The coronavirus spike protein variant according to Item (1-2), comprising (b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 44, 45, 56, 65, 74, 75, 78, 110, 111, 113, 115, 116, 117, 118, 170, 171, 173, and 175 in the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32, or
(c) an RBD consisting of an amino acid sequence obtained by substituting, deleting, inserting, or adding amino acids at positions other than positions 44, 45, 56, 65, 74, 75, 78, 110, 111, 113, 115, 116, 117, 118, 170, 171, 173, and 175 in the amino acid sequence set forth in SEQ ID NO: 32, and having at least 95% sequence identity (preferably at least 96%, at least 97%, at least 98%, or at least 99% sequence identity) to the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32.
(1-4) A coronavirus spike protein variant comprising an RBD co nsisting of an amino acid sequence consisting of amino acid re sidues at positions 325 to 527 of an amino acid sequence set f orth in any one of SEQ ID NOs: 65, 68, 71, 74, 76, 82, 84, 87, 89, 92, 93, 94, 96, 97, 100, 102 to 104, and 106 to 110.
(2) The spike protein variant according to Item (1) or (1-1), wherein the amino acid sequence set forth in SEQ ID NO: 1 is an amino acid sequence set forth in any one of SEQ ID NOs: 2 to 5.
(3) The spike protein variant according to any one of the Items (1), (2), and (1-1) to (1-4), comprising an amino acid sequence having at least 90% sequence identity (preferably at least 91%, at least 92%, at least 93%, at least 94%, at least 95%, at least 96%, at least 97%, at least 98%, or at least 99% sequence identity) to an amino acid sequence consisting of amino acid residues at positions 14 to 1211 of the amino acid sequence set forth in SEQ ID NO: 6.
(4) The spike protein variant according to any one of Items (1) to (3) and (1-1) to (1-4), comprising an N-terminal domain consisting of: (a) an amino acid sequence set forth in SEQ ID NO: 7;
(b) an amino acid sequence obtained by substituting, deleting, inserting, or adding one to several amino acids (preferably 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 7; or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 7.

(5) The spike protein variant according to Item (4), wherein the amino acid sequence set forth in SEQ ID NO: 7 is an amino acid sequence set forth in either SEQ ID NO: 8 or 9.
(5-1) The spike protein variant according to any one of Items (1) to (3) and (1-1) to (1-4), comprising an NTD consisting of: (a) an amino acid sequence of a region corresponding to spike protein NTD derived from a betacoronavirus (preferably from sarbecovirus);
(b) an amino acid sequence obtained by substituting, deleting, inserting, or adding one to several amino acids (preferably 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in an amino acid sequence of the region corresponding to the spike protein NTD derived from a betacoronavirus (preferably a sarbecovirus); or
(c) an amino acid sequence having at least 90% sequence identity (preferably at least 91%, at least 92%, at least 93%, at least 94%, at least 95%, at least 96%, at least 97%, at least 98%, or at least 99% sequence identity) to an amino acid sequence of a region corresponding to the spike protein NTD derived from a betacoronavirus (preferably from sarbecovirus).

(5-2) The spike protein variant according to Item (5-1), wherein the betacoronavirus is a sarbecovirus selected from viruses according to the table below.

**[Table 1]**

| Virus Name (Lineage) | GISAID ID or GenBank ID |
|---|---|
| CH.1. | EPI_ISL_15760478 |
| BQ.1.18 | EPI_ASL_15508450 |
| XBB.1.16 | EPI_ISL_16835403 |
| XBB.1.16.1 | EPI_ISL_17030043 |
| XBF | EPI_ISL_16611536 |
| XBB.2.3 | EPI_ISL_16382405 |
| XBB.2.3.2 | EPI_ISL_17206421 |
| EG.5.1 | EPI_ISL_17471668 |
| BA.2.86 | EPI_ISL_18114953 |
| JN. 1 | EPI_ISL_18428109 |
| KP.2 | EPI_ISL_18957516 |
| KP.3 | EPI_ISL_10-59439 |
| WIV-1 | KF367457.1 |
| RaTG13 | QHR63300.2 |
| SHC014 | AGZ48806.1 |
| Pangolin-GD | WCZ55842.1 |
| Pangolin-GX | QVT76606.1 |
| HKU3 | QND76034.1 |

(5-3) The spike protein variant according to Item (5-1), wherein the betacoronavirus is Middle East respiratory syndrome coronavirus (MERS coronavirus, MERS-CoV).
(5-4) The spike protein variant according to any one of Items (1) to (5) and (1-1) to (1-4), comprising an NTD consisting of an amino acid sequence consisting of amino acid residues at positions 14 to 301 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.
(6) The spike protein variant according to any one of Items (1) to (5), (1-1) to (1-4), and (5-1) to (5-4), comprising a region consisting of SD1, SD2, and S2 subunits, the region consisting of: (a) an amino acid sequence set forth in SEQ ID NO: 10;
(b) an amino acid sequence obtained by substituting, deleting, inserting, or adding one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 10; or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 10.

(7) The spike protein variant according to Item (6), wherein the amino acid sequence set forth in SEQ ID NO: 10 is an amino acid sequence set forth in any one of SEQ ID NOs: 11 to 15.
(7-1) The spike protein variant according to any one of Items (1) to (6), (1-1) to (1-4), and (5-1) to (5-4), comprising a region consisting of SD1, SD2 and S2 subunits, the region consisting of an amino acid sequence consisting of amino acid residues at positions 528 to 1209 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.
(8) The spike protein variant according to any one of Items (1) to (7), (1-1) to (1-4), (5-1) to (5-4), and (7-1), comprising a region consisting of SD1, SD2 and S2 subunits, the region consisting of: (a) an amino acid sequence set forth in SEQ ID NO: 16;
(b) an amino acid sequence obtained by substituting, deleting, inserting, or adding one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 16; or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 16.

(9) The spike protein variant according to Item (8), having amino acid substitutions corresponding to Q434C substitution and S472C substitution in the amino acid sequence set forth in SEQ ID NO: 16.
(10) The spike protein variant according to Item (8) or (9), having amino acid substitutions corresponding to K455P substitution and/or V456P substitution in the amino acid sequence set forth in SEQ ID NO: 16.
(11) The spike protein variant according to any one of Items (8) to (10), having amino acid substitutions corresponding to one or more amino acid substitutions selected from the group consisting of F286P substitution, A361P substitution, A368P substitution, and A411P substitution in the amino acid sequence set forth in SEQ ID NO: 16.
(12) The spike protein variant according to any one of Items (8) to (11), having an amino acid substitutions corresponding to T201P substitution and/or K264P substitution in the amino acid sequence set forth in SEQ ID NO: 16.
(13) The spike protein variant according to any one of Items (8) to (12), wherein one or more amino acid residues corresponding to RRAR (SEQ ID NO: 17) at positions 151 to 154 of the amino acid sequence set forth in SEQ ID NO: 16 have amino acid mutations and are not cleaved by a furin protease.
(14) The spike protein variant according to Item (13), wherein amino acid residues corresponding to RRAR at positions 151 to 154 of the amino acid sequence set forth in SEQ ID NO: 16 are substituted with GSAS (SEQ ID NO: 18).
(15) The spike protein variant according to any one of Items (8) to (14), having amino acid substitutions corresponding to one or more amino acid substitutions selected from the group consisting of N543Q substitution, N567Q substitution, and N642L substitution in the amino acid sequence set forth in SEQ ID NO: 16.
(16) The spike protein variant according to any one of Items (1) to (15), (1-1) to (1-4), (5-1) to (5-4), and (7-1), lacking a transmembrane domain and a cytoplasmic tail.
(17) The spike protein variant according to Item (16), wherein a trimerization domain is added to the C-terminus.
(17-1) The spike protein variant according to Item (17), wherein the trimerization domain is a foldon sequence.
(18) The spike protein variant according to any one of Items (1) to (17), (1-1) to (1-4), (5-1) to (5-4), (7-1), and (17-1), wherein a His tag is added at C-terminus.
(19) The spike protein variant according to any one of Items (1) to (18), (1-1), (5-1), and (17-1), comprising an amino acid sequence consisting of amino acid residues at positions from position 14 to the C-terminus of an amino acid sequence set forth in any one of SEQ ID NOs: 21 to 27.
(19-1) The spike protein variant according to any one of Items (1) to (19) and (17-1), comprising an amino acid sequence con sisting of amino acid residues at positions 14 to 1236 of an a mino acid sequence set forth in any one of SEQ ID NOs: 21 to 2 7.
(19-2) The spike protein variant according to any one of Items (1) to (19), (1-1), (5-1) to (5-4), (7-1), and (17-1), comprising (a) an amino acid sequence obtained by substituting, deleting, inserting, or adding one to several amino acids (preferably 1 to 60, 1 to 50, 1 to 40, 1 to 30, 1 to 20, 1 to 15, 1 to 10, 1 to 9, 1 to 8, 1 to 7, 1 to 6, 1 to 5, 1 to 4, 1 to 3, or 1 to 2 amino acids) in an amino acid sequence consisting of amino acid residues at positions 14 to 1236 of the amino acid sequence set forth in any one of SEQ ID NOs: 21 to 27; or
(b) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence consisting of amino acid residues at positions 14 to 1236 of the amino acid sequence set forth in any one of SEQ ID NOs: 21 to 27.
(19-3) The spike protein variant according to Item (19-2), having neutralizing antibody-inducing activity against SARS-CoV-2 and SARS-CoV-1 equivalent to that of a spike protein variant comprising an amino acid sequence consisting of amino acid residues at positions 14 to 1236 of the amino acid sequence set forth in any one of SEQ ID NOs: 21 to 27.
(19-4) The spike protein variant according to Item (19-2) or (19-3), comprising an amino acid sequence consisting of amino acid residues at positions 14 to 1236 of the amino acid sequence set forth in any one of SEQ ID NOs: 65 to 122.
(19-5) The spike protein variant according to any one of Items (1) to (19), (1-1), (1-4), (5-1), (5-4), (7-1), and (17-1), comprising an amino acid sequence consisting of amino acid residues at positions 14 to 1209 of the amino acid sequence set forth in any one of SEQ ID NOs: 21 to 27.
(19-6) The spike protein variant according to any one of Items (1) to (19), (1-1), (5-1) to (5-4), (7-1), and (17-1), comprising: (a) an amino acid sequence obtained by substituting, deleting, inserting, or adding one to several amino acids (preferably 1 to 60, 1 to 50, 1 to 40, 1 to 30, 1 to 20, 1 to 15, 1 to 10, 1 to 9, 1 to 8, 1 to 7, 1 to 6, 1 to 5, 1 to 4, 1 to 3, or 1 to 2 amino acids) in an amino acid sequence consisting of amino acid residues at positions 14 to 1209 of an amino acid sequence set forth in any one of SEQ ID NOs: 21 to 27; or
(b) an amino acid sequence having at least 90% sequence identity (preferably at least 91%, at least 92%, at least 93%, at least 94%, at least 95%, at least 96%, at least 97%, at least 98%, or at least 99% sequence identity) to an amino acid sequence consisting of amino acid residues at positions 14 to 1209 of the amino acid sequence set forth in any one of SEQ ID NOs: 21 to 27.
(19-7) The spike protein variant according to Item (19-6), having neutralizing antibody-inducing activity against SARS-CoV-2 and SARS-CoV-1 equivalent to that of a spike protein variant comprising an amino acid sequence consisting of amino acid residues at positions 14 to 1209 of the amino acid sequence set forth in any one of SEQ ID NOs: 21 to 27.
(19-8) The spike protein variant according to Item (19-6) or (19-7), comprising an amino acid sequence consisting of amino acid residues at positions 14 to 1209 of the amino acid sequence set forth in any one of SEQ ID NOs: 65 to 122.
(20) A coronavirus spike protein variant having at least 99% s equence identity (preferably at least 99.3%, at least 99.4%, a t least 99.5%, at least 99.6%, at least 99.7%, at least 99.9%, or at least 99.9% sequence identity) to an amino acid sequenc e consisting of amino acid residues at positions 14 to 1236 of an amino acid sequence set forth in SEQ ID NO: 26, and compri sing amino acid sequences of amino acid residues at positions 14 to 324 and positions 528 to 1236 of an amino acid sequence set forth in SEQ ID NO: 26.
(20-1) A coronavirus spike protein variant comprising an amino acid sequence obtained by substituting, deleting, inserting, or adding one to several amino acids at positions 14 to 1236 of the amino acid sequence set forth in SEQ ID NO: 26, wherein the amino acid sequence comprises amino acid residues at positions 14 to 324 and positions 528 to 1236 of the amino acid sequence set forth in SEQ ID NO: 26.
(20-2) The spike protein variant according to Item (20) or (20 -1), having neutralizing antibody-inducing activity against SA RS-CoV-2 and SARS-CoV-1 equivalent to that of a spike protein variant comprising an amino acid sequence of consisting amino acid residues at positions 14 to 1236 of the amino acid sequen ce set forth in SEQ ID NO: 26.
(21) The spike protein variant according to Item (20) or (20-1), comprising an amino acid sequence consisting of amino acid residues at positions 14 to 1236 of an amino acid sequence set forth in any one of SEQ ID NOs: 65, 68, 71, 74, 76, 82, 84, 87, 89, 92, 93, 94, 96, 97, 100, 102 to 104, and 106 to 110.
(22) A coronavirus spike protein variant having at least 99% s equence identity (preferably at least 99.3%, at least 99.4%, a t least 99.5%, at least 99.6%, at least 99.7%, at least 99.9%, or at least 99.9% sequence identity) to an amino acid sequenc e consisting of amino acid residues at positions 14 to 1236 of an amino acid sequence set forth in SEQ ID NO: 26, and compri sing an amino acid sequence of consisting amino acid residues at positions 325 to 527 of an amino acid sequence set forth in SEQ ID NO: 26.
(22-1) A coronavirus spike protein variant comprising an amino acid sequence obtained by substituting, deleting, inserting, or adding one to several amino acids at positions 14 to 1236 of the amino acid sequence set forth in SEQ ID NO: 26, wherein the amino acid sequence comprises amino acid residues at positions 325 to 527 of the amino acid sequence set forth in SEQ ID NO: 26.
(22-2) The spike protein variant according to Item (22) or (22-1), having neutralizing antibody-inducing activity against SARS-CoV-2 and SARS-CoV-1 equivalent to that of a spike protein variant comprising an amino acid sequence consisting of amino acid residues at positions 14 to 1236 of the amino acid sequence set forth in SEQ ID NO: 26.
(23) The spike protein variant according to Item (22) or (22-1), comprising an amino acid sequence consisting of amino acid residues at positions 14 to 1236 of the amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.
(23-1) A coronavirus spike protein variant comprising:
an RBD consisting of an amino acid sequence of the following (a) or (b):
   (a) an amino acid sequence set forth in SEQ ID NOs: 1 to 5; or
   (b) an amino acid sequence consisting of amino acid residues at positions 325 to 527 of an amino acid sequence set forth in any one of SEQ ID NOs: 65, 68, 71, 74, 76, 82, 84, 87, 89, 92, 93, 94, 96, 97, 100, 102 to 104, and 106 to 110,
an NTD consisting of an amino acid sequence of the following (c) or (d):
   (c) an amino acid sequence set forth in SEQ ID NOs: 7 to 9; or
   (d) an amino acid sequence consisting of amino acid residues at positions 14 to 301 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122,
a region consisting of SD1, SD2 and S2 subunits consisting of an amino acid sequence of the following (e) or (f):
   (e) an amino acid sequence set forth in SEQ ID NOs: 7 to 9; or
   (f) an amino acid sequence consisting of amino acid residues at positions 528 to 1209 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.

(24) An immunogenic composition comprising the spike protein variant according to any one of Items (1) to (23), (1-1) to (1-4), (5-1) to (5-4), (7-1), (17-1), (19-1) to (19-8), (20-1), (20-2), (22-1), (22-2), and (23-1).
(25) The immunogenic composition according to Item (24) comprising only one type of the spike protein variant set forth in any one of Items (1) to (23), (1-1) to (1-4), (5-1) to (5-4), (7-1), (17-1), (19-1) to (19-8), (20-1), (20-2), (22-1), (22-2), and (23-1) as an antigen derived from a coronavirus spike protein, and not comprising any other antigen derived from the coronavirus spike protein.
(26) The immunogenic composition according to Item (24) or (25), further comprising an adjuvant.
(27) The immunogenic composition according to Item (26), wherein the adjuvant is an adjuvant comprising squalene, tocopherol, and polysorbate 80.
(28) A nucleic acid encoding the spike protein variant according to any one of Items (1) to (23), (1-1) to (1-4), (5-1) to (5-4), (7-1), (17-1), (19-1) to (19-8), (20-1), (20-2), (22-1), (22-2), and (23-1).
(29) An expression vector comprising the nucleic acid according to Item (28).
(30) A method of preventing an infection with SARS-CoV-2 and/or other coronavirus (preferably betacoronavirus, more preferably sarbecovirus), the method comprising administering to an individual an effective amount of the immunogenic composition according to any one of Items (24) to (27), the nucleic acid according to Item (28), or the expression vector according to Item (29).
(31) The immunogenic composition according to any one of Items (24) to (27), the nucleic acid according to Item (28), or the expression vector according to Item (29), for preventing an infection with SARS-CoV-2 and/or other coronavirus (preferably betacoronavirus, more preferably sarbecovirus).
(32) Use of the immunogenic composition according to any one of Items (24) to (27), the nucleic acid according to Item (28), or the expression vector according to Item (29) for the manufacture of a pharmaceutical for prevention of an infection with SARS-CoV-2 and/or other coronavirus (preferably betacoronavirus, more preferably sarbecovirus).
(33) A spike protein variant comprising an amino acid sequence consisting of amino acid residues at positions 14 to 1209 of an amino acid sequence set forth in any one of SEQ ID NOs: 21 to 27 and 65 to 122.
(34) The spike protein variant according to Item (33), comprising an amino acid sequence consisting of amino acid residues at positions 14 to 1236 of the amino acid sequence set forth in any one of SEQ ID NOs: 21 to 27 and SEQ ID NOs: 65 to 122.
(35) The spike protein variant according to Item (34), comprising an amino acid sequence consisting of amino acid residues at position 14 to the C-terminus of the amino acid sequence set forth in any one of SEQ ID NOs: 21 to 27 and SEQ ID NOs: 65 to 122.
(36) An immunogenic composition comprising the spike protein variant according to any one of Items (33) to (35).
(37) The immunogenic composition according to Item (36), further comprising only one type of spike protein variant according to any one of Items (33) to (35) as an antigen derived from a coronavirus spike protein, and not comprising any other antigen derived from the coronavirus spike protein.
(38) The immunogenic composition according to Item (36) or (37), further comprising an adjuvant.
(39) The immunogenic composition according to Item (37), wherein the adjuvant is an adjuvant comprising squalene, tocopherol, and polysorbate 80.
(40) A nucleic acid encoding the spike protein variant according to any one of Items (33) to (35).
(41) An expression vector comprising the nucleic acid according to Item (40).
(42) The immunogenic composition according to any one of Items (36) to (39), the nucleic acid according to Item (40), or the expression vector according to Item (41), for preventing an infection with SARS-CoV-2 and/or other coronavirus (preferably betacoronavirus, more preferably sarbecovirus).

### [EFFECT OF THE INVENTION]

The coronavirus spike protein variants according to the present invention exhibit excellent effects in that a monovalent antigen can elicit broad immunogenicity.

### [BRIEF DESCRIPTION OF THE DRAWINGS]

[Fig. 1]

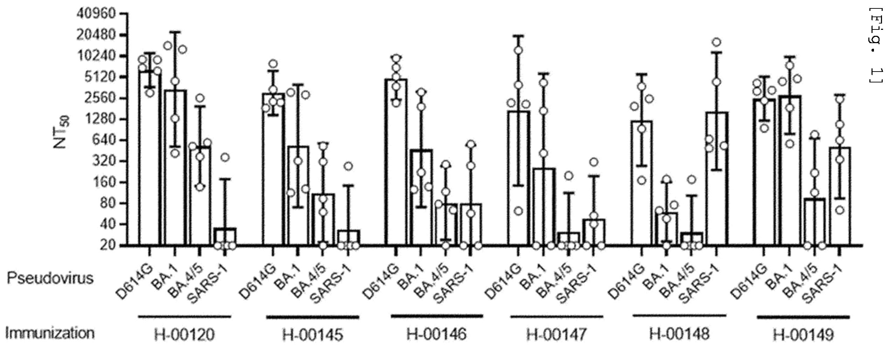

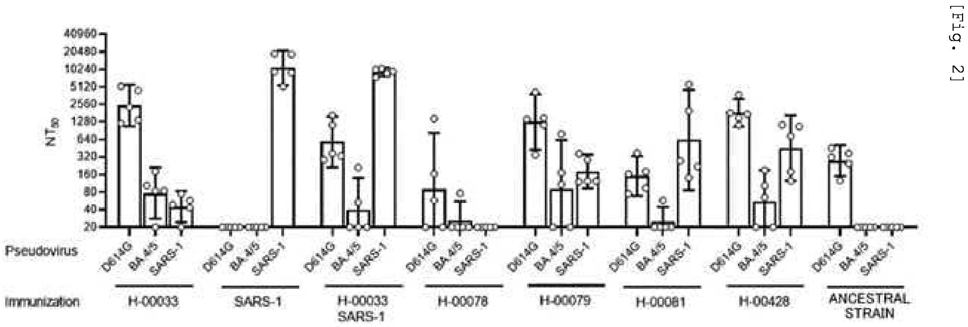

   Fig. 1 shows the results of neutralizing activity assay on each pseudovirus of mouse serum immunized with each antigen, performed in Example 5. In Fig. 1, "Pseudovirus" indicates the virus name (lineage) of the pseudovirus, and "Immunization" indicates the type of antigen used for immunization. In Fig. 1, the vertical axis represents the neutralizing antibody titer (NT50), the vertical bar represents the geometric mean value for each combination of antigen and pseudovirus within each group, the error bar represents 95% confidence interval, and the small circle represents individual neutralizing antibody titer for each group (the same applies to Figs. 2 to 24).
[Fig. 2]
   Fig. 2 shows the results of neutralizing activity assay on each pseudovirus of mouse serum immunized with each antigen, performed in Example 6.
[Fig. 3]
   Fig. 3 shows the results of neutralizing activity assay on each pseudovirus of mouse serum immunized with each antigen, performed in Example 7.
[Fig. 4]
   Fig. 4 shows the results of neutralizing activity assay on each pseudovirus of mouse serum immunized with each antigen, performed in Example 8.
[Fig. 5]

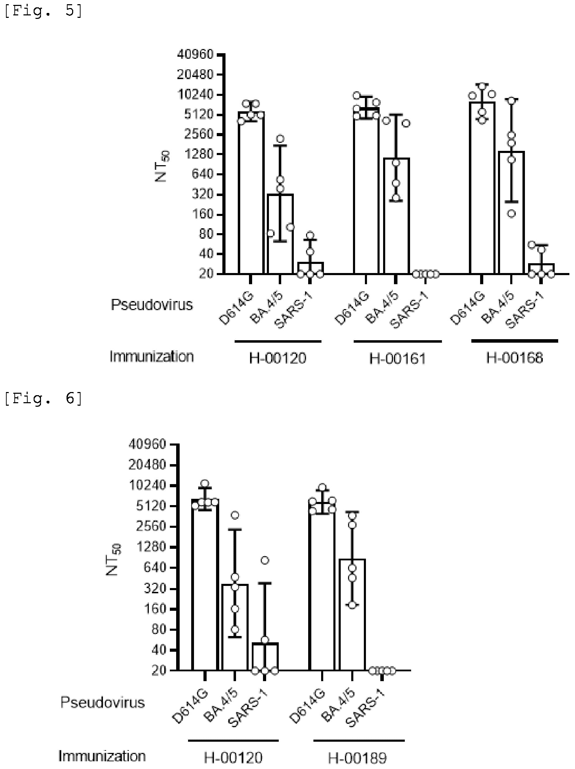

   Fig. 5 shows the results of neutralizing activity assay on each pseudovirus of mouse serum immunized with each antigen, performed in Example 9.
[Fig. 6]
   Fig. 6 shows the results of neutralizing activity assay on each pseudovirus of mouse serum immunized with each antigen, performed in Example 10.
[Fig. 7]

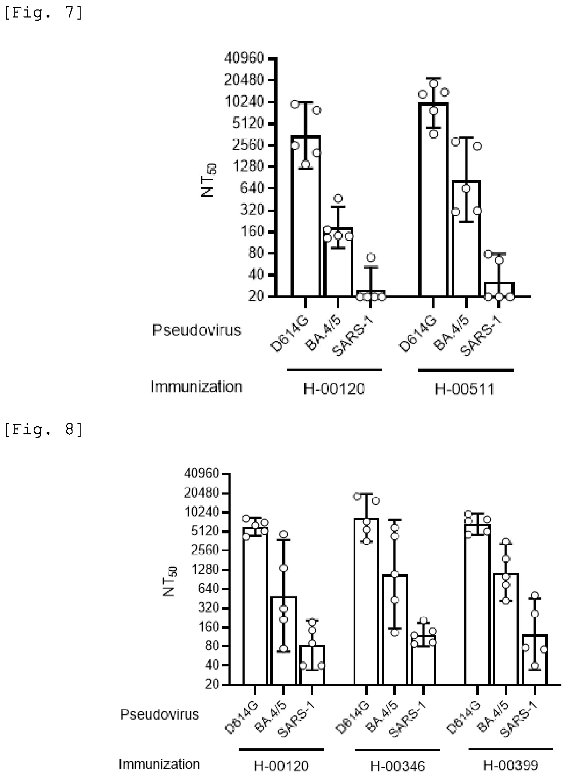

   Fig. 7 shows the results of neutralizing activity assay on each pseudovirus of mouse serum immunized with each antigen, performed in Example 11.
[Fig. 8]
   Fig. 8 shows the results of neutralizing activity assay on each pseudovirus of mouse serum immunized with each antigen, performed in Example 12.
[Fig. 9]

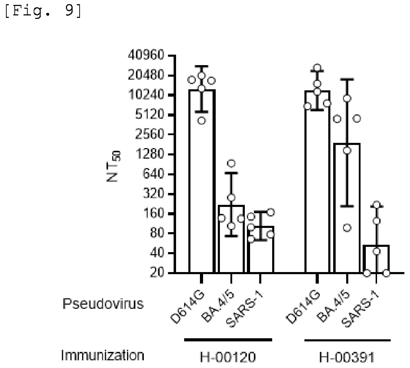

   Fig. 9 shows the results of neutralizing activity assay on each pseudovirus of mouse serum immunized with each antigen, performed in Example 13.
[Fig. 10]

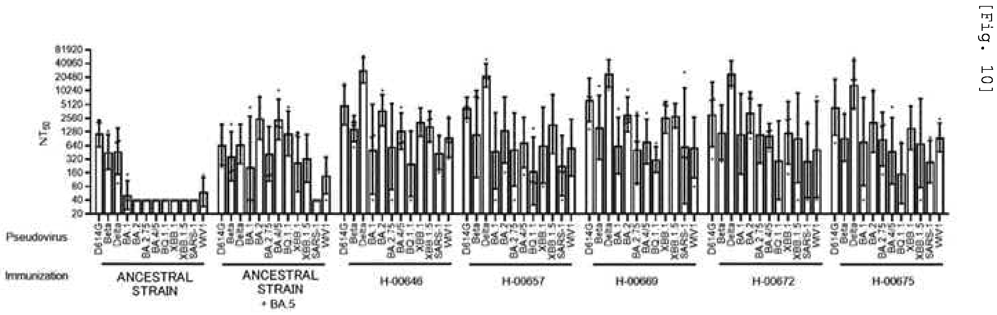

   Fig. 10 shows the results of neutralizing activity assay on each pseudovirus of mouse serum immunized with each antigen, performed in Example 14.
[Fig. 11]

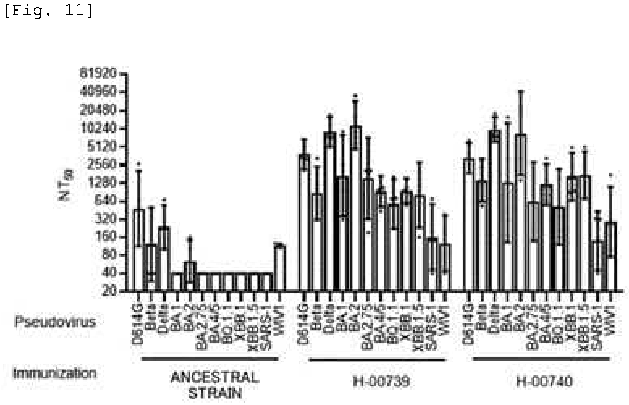

   Fig. 11 shows the results of neutralizing activity assay on each pseudovirus of mouse serum immunized with each antigen, performed in Example 15.
[Fig. 12]

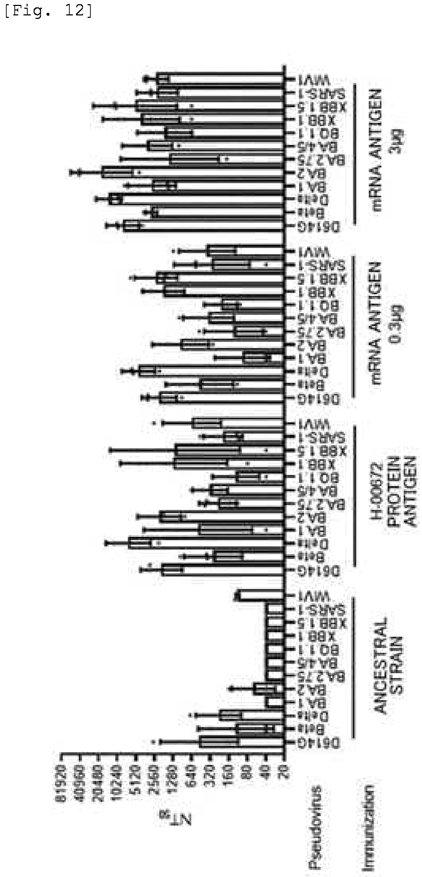

   Fig. 12 shows the results of neutralizing activity assay on each pseudovirus of mouse serum immunized with each antigen, performed in Example 16.
[Fig. 13]

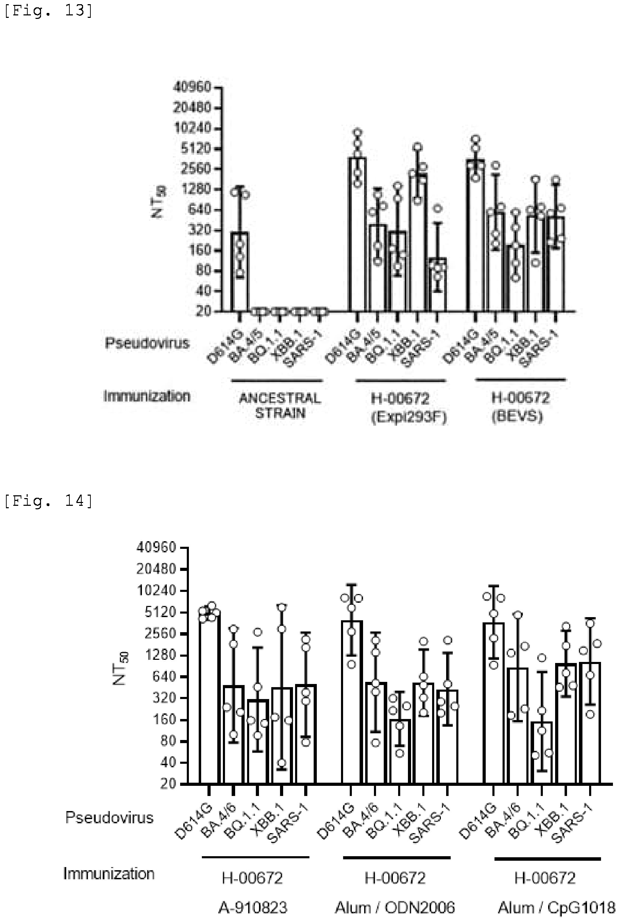

   Fig. 13 shows the results of neutralizing activity assay on each pseudovirus of mouse serum immunized with each antigen, performed in Example 17.
[Fig. 14]
   Fig. 14 shows the results of neutralizing activity assay on each pseudovirus of mouse serum immunized with each antigen, performed in Example 18.
[Fig. 15]

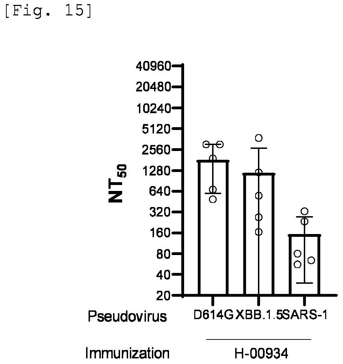

   Fig. 15 shows the results of neutralizing activity assay on each pseudovirus of mouse serum immunized with each antigen, performed in Example 19.
[Fig. 16]

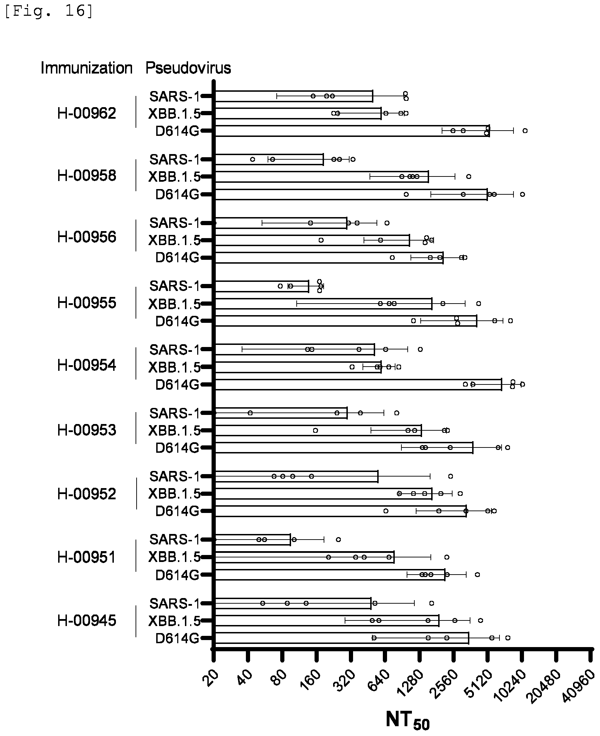

   Fig. 16 shows the results of neutralizing activity assay on each pseudovirus of mouse serum immunized with each antigen, performed in Example 19.
[Fig. 17]

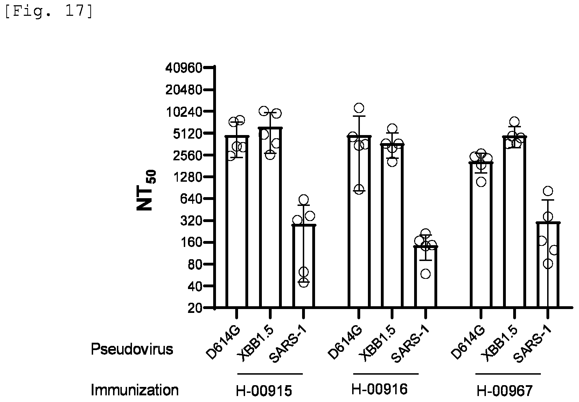

   Fig. 17 shows the results of neutralizing activity assay on each pseudovirus of mouse serum immunized with each antigen, performed in Example 19.
[Fig. 18]

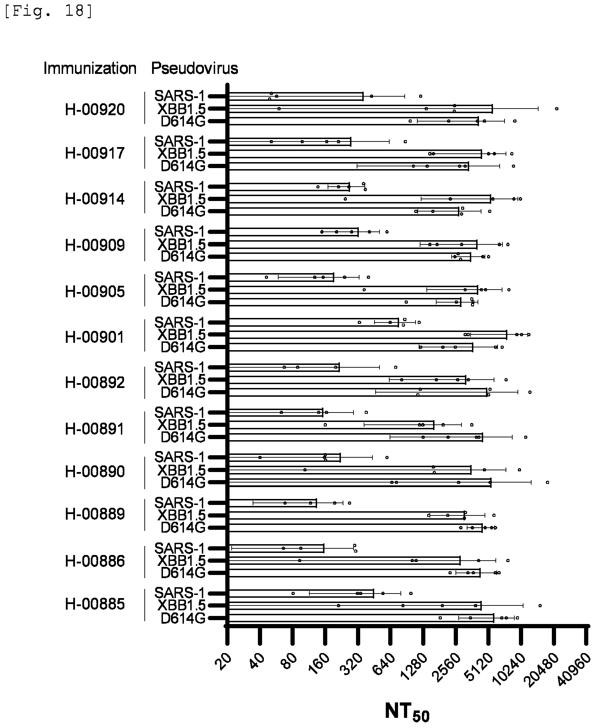

   Fig. 18 shows the results of neutralizing activity assay on each pseudovirus of mouse serum immunized with each antigen, performed in Example 19.
[Fig. 19]

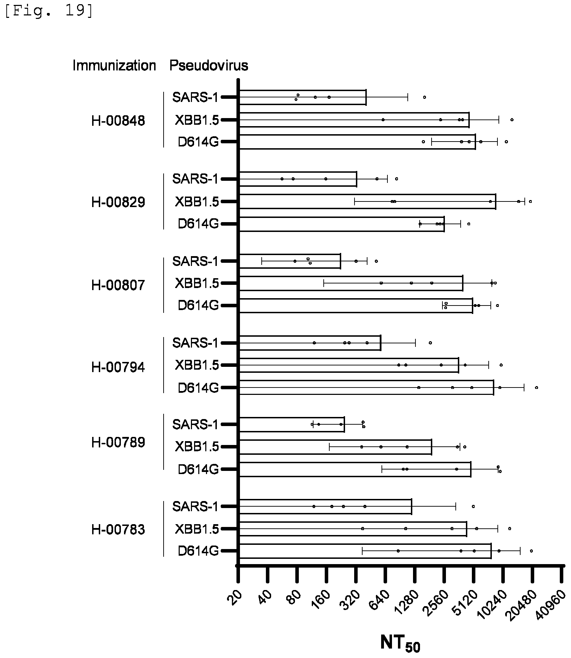

   Fig. 19 shows the results of neutralizing activity assay on each pseudovirus of mouse serum immunized with each antigen, performed in Example 19.
[Fig. 20]

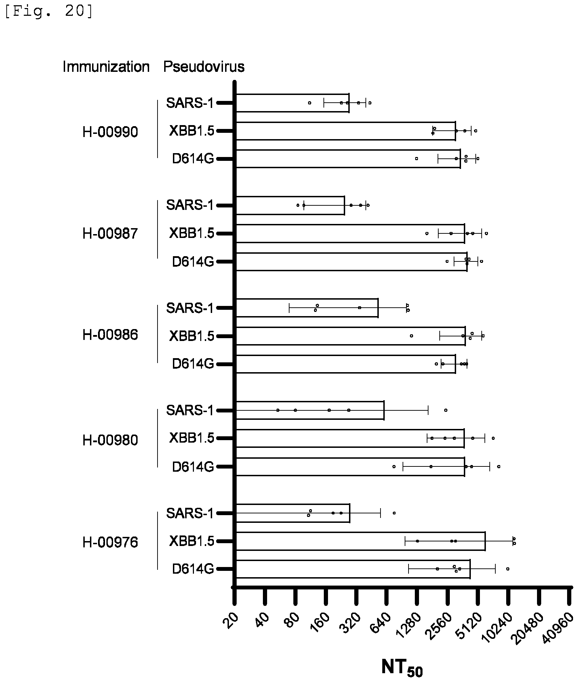

   Fig. 20 shows the results of neutralizing activity assay on each pseudovirus of mouse serum immunized with each antigen, performed in Example 19.
[Fig. 21]

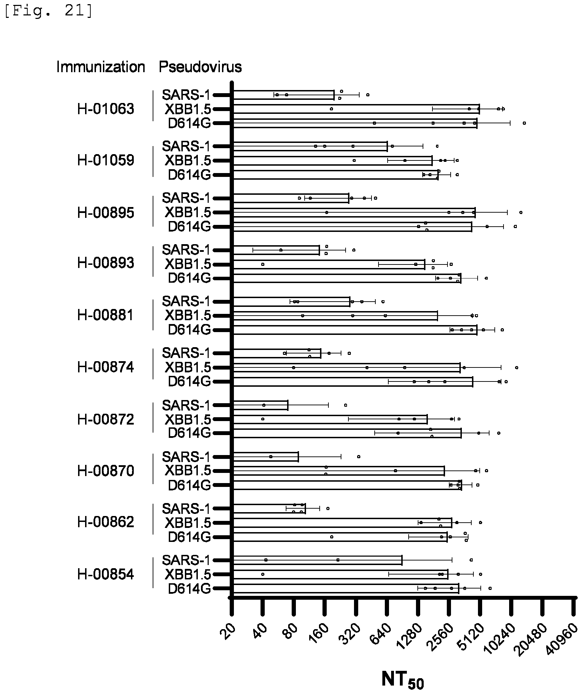

   Fig. 21 shows the results of neutralizing activity assay on each pseudovirus of mouse serum immunized with each antigen, performed in Example 19.
[Fig. 22]

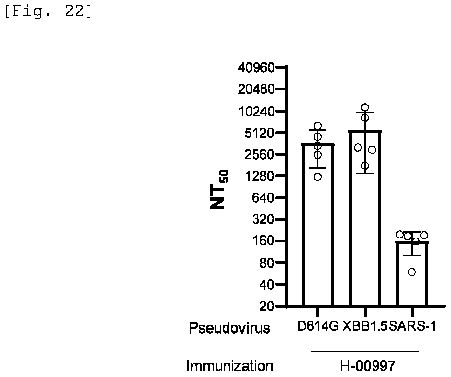

   Fig. 22 shows the results of neutralizing activity assay on each pseudovirus of mouse serum immunized with each antigen, performed in Example 19.
[Fig. 23]

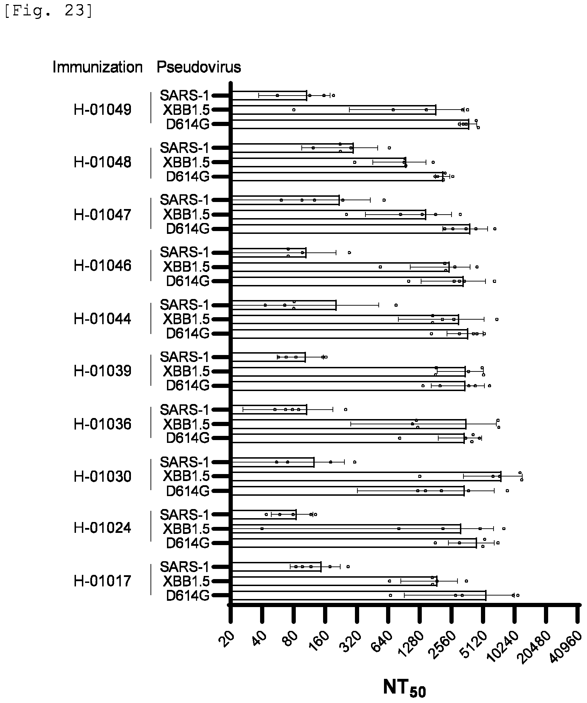

   Fig. 23 shows the results of neutralizing activity assay on each pseudovirus of mouse serum immunized with each antigen, performed in Example 19.
[Fig. 24]

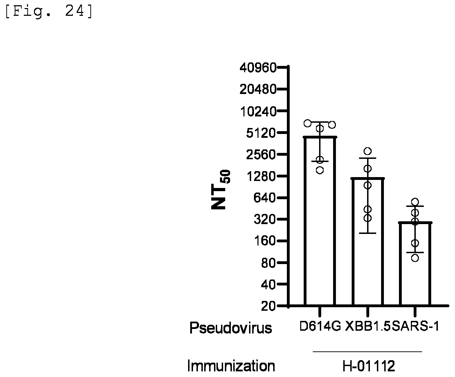

   Fig. 24 shows the results of neutralizing activity assay on each pseudovirus of mouse serum immunized with each antigen, performed in Example 19.

### [MODE FOR CARRYING OUT THE INVENTION]

The term "consist of" means having only the constituent elements. The term "comprise" means that elements are not limited to the constituent elements, and elements that are not described are not excluded.

The present invention will be described below with reference to embodiments. Throughout this specification, it should be understood that the representation of the singular also includes the concept of the plural unless stated otherwise. Thus, unless stated otherwise, singular articles (e.g., "a", "an", "the", and the like in English) can be understood to include the concepts of the plural forms. Unless otherwise defined, it should be understood that the terms used in the present specification are used in a meaning normally used in the art. Accordingly, unless otherwise defined, all terminologies and scientific and technical terms used in the present specification have the same meanings as commonly understood by those having ordinary skill in the art to which the present invention belongs. In the event of any inconsistency, the present specification (including the definitions) shall prevail.

The "sequence identity" with respect to a reference polypeptide sequence is defined as the percentage of amino acid residues in a candidate sequence that are identical to the amino acid residues in the reference polypeptide, after aligning the sequences and introducing gaps if necessary to achieve maximum percent sequence identity, and without considering any conservative substitutions as part of the sequence identity. For the purpose of determining sequence identity, the alignment may be achieved by various methods within the skill of those in the art, for example, by using publicly available computer software such as BLAST.

As used herein, "ancestral strain spike protein" refers to the spike protein of SARS-CoV-2 as described in NCBI Reference No.: YP_009724390.1.

As used in the present invention, the "immunogenicity" refers to the property of stimulating the immune system of a subject to elicit an immunoreaction, such as the production of antibodies against a target pathogen. As used herein, the "immunogenic composition" denotes a composition that induces an immune response in a subject upon administration.

As used herein, the "neutralizing antibody-inducing activity" refers to the ability of an antigen protein to induce neutralizing antibodies, which can be evaluated by measuring the neutralizing antibody titer in immune serum obtained by administering the antigen protein to a test animal. The neutralizing activity of the serum can be assessed by the serum dilution required to reduce the number of infected test viruses by 50%, i.e., the neutralizing antibody titer (NT50).

As used herein, a spike protein variant "having neutralizing antibody-inducing activity against SARS-CoV-2 and SARS-CoV-1 equivalent to" refers to a spike protein variant which has both a neutralizing antibody titer of at least 12.5% (preferably 25%, 50%, 70%, 80%, 90%, 95%, or 100% or more) of the neutralizing antibody titer against the spike protein of the SARS-CoV-2 D614G lineage (GISAID ID: EPI_ISL_529135; hereinafter also referred to as SARS-CoV-2 (D614G)) and a neutralizing antibody titer of at least 12.5% (preferably 25%, 50%, 70%, 80%, 90%, 95%, or 100% or more) of the neutralizing antibody titer against SARS-CoV-1 (GenBank ID: AY278741.1). The neutralizing antibody-inducing activity of a spike protein variant can be confirmed, for example, by producing the spike protein variant to be measured in accordance with the method described in Example 1, and measuring the neutralizing antibody titer in serum obtained by immunizing mice in accordance with the method described in Example 4.

The present invention relates to coronavirus spike protein variants. As used herein, the "coronavirus spike protein variant" means a spike protein having one or more amino acid mutations relative to the amino acid sequence of a naturally occurring coronavirus spike protein, and may be simply referred to as a "spike protein variant". As used herein, the "amino acid mutation" refers to a substitution, deletion, insertion, or addition of one or more amino acids, or a combination thereof.

The spike protein variant according to the present invention comprises:
(a) a receptor binding domain (RBD) consisting of an amino acid sequence set forth in SEQ ID NO: 1;
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1; or
(c) an RBD consisting of an amino acid sequence that has at least 90% sequence identity (preferably at least 91%, at least 92%, at least 93%, at least at least 94%, at least 95%, at least 96%, at least 97%, at least 98%, or at least 99% sequence identity) to the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1.

The receptor binding domain (RBD) refers to a region of a coronavirus spike protein that contributes to binding to a receptor on a cell; however, in the present invention, the term "receptor binding domain (RBD)" is used to specify a region within the spike protein and does not require having binding activity to a receptor on a cell. In the ancestral strain spike protein, the amino acid sequence consisting of amino acid residues at positions 329 to 531 corresponds to RBD.

The amino acid sequence set forth in SEQ ID NO: 1 includes the amino acid sequences set forth in any one of SEQ ID NOs: 2 to 5.

The present inventors created various hybrid RBDs based on the RBD sequences of SARS-CoV-2 and SARS-CoV-1 in order to design an RBD sequence having neutralizing antibody-inducing activity against both SARS-CoV-2 and SARS-CoV-1, identified the RBD sequence of SEQ ID NO: 3, and further obtained, as variations thereof, the RBD sequences of SEQ ID NOs: 2, 4, and 5.

SEQ ID NO: 1 is an amino acid sequence (an RBD consensus sequence) obtained by generalizing the amino acid sequences of RBDs (SEQ ID NOs: 2 to 5) contained in the spike protein variant according to the present invention described below, and the amino acid sequences are as follows.

**[Table 2]**

| Name | Amino Acid Sequence |
|---|---|
| RBD Consensus Sequence | FPXITNLCPFGEVFNATKFPSVYAWERKKISNCVADYSVLYNST FFSTFKCYGVSATKLNDLCFSNVYADSFVVKGDDVRQIAPGQTG VIADYNYKLPDDFMGCVIAWNSNNLDSKVGGNYNYRYRLFRKSX LKPFERDISTEIYQAGXKPCNGVEGFNCYFPLQSYGFQPTXGVG YQPYRVVVLSFELLHAPATVCGPKKST(SEQ ID NO: 1) |
| RBD Sequence 1 (H-00646, H-00669, H-00672, H-00675) | FPQITNLCPFGEVFNATKFPSVYAWERKKISNCVADYSVLYNST FFSTFKCYGVSATKLNDLCFSNVYADSFVVKGDDVRQIAPGQTG VIADYNYKLPDDFMGCVIAWNSNNLDSKVGGNYNYRYRLFRKSN LKPFERDISTEIYQAGSKPCNGVEGFNCYFPLQSYGFQPTNGVG YQPYRVVVLSFELLHAPATVCGPKKST(SEQ ID NO: 2) |
| RBD Sequence 2 (H-00657) | FPNITNLCPFGEVFNATKFPSVYAWERKKISNCVADYSVLYNST FFSTFKCYGVSATKLNDLCFSNVYADSFVVKGDDVRQIAPGQTG VIADYNYKLPDDFMGCVIAWNSNNLDSKVGGNYNYRYRLFRKSN LKPFERDISTEIYQAGSKPCNGVEGFNCYFPLQSYGFQPTNGVG YQPYRVVVLSFELLHAPATVCGPKKST(SEQ ID NO: 3) |
| RBD Sequence 3 (H-00739) | FPQITNLCPFGEVFNATKFPSVYAWERKKISNCVADYSVLYNST FFSTFKCYGVSATKLNDLCFSNVYADSFVVKGDDVRQIAPGQTG VIADYNYKLPDDFMGCVIAWNSNNLDSKVGGNYNYRYRLFRKSK LKPFERDISTEIYQAGSKPCNGVEGFNCYFPLQSYGFQPTYGVG YQPYRVVVLSFELLHAPATVCGPKKST(SEQ ID NO: 4) |
| RBD Sequence 4 (H-00740) | FPQITNLCPFGEVFNATKFPSVYAWERKKISNCVADYSVLYNST FFSTFKCYGVSATKLNDLCFSNVYADSFVVKGDDVRQIAPGQTG VIADYNYKLPDDFMGCVIAWNSNNLDSKVGGNYNYRYRLFRKSK LKPFERDISTEIYQAGNKPCNGVEGFNCYFPLQSYGFQPTYGVG YQPYRVVVLSFELLHAPATVCGPKKST(SEQ ID NO: 5) |

In the amino acid sequence of SEQ ID NO: 1, X at position 3 is Q or N, X at position 132 is N or K, X at position 149 is S or Y, and X at position 173 is N or Y.

The amino acid sequence set forth in SEQ ID NO: 1 includes the amino acid sequences set forth in any one of SEQ ID NOs: 2 to 5.

The (b) RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1 includes an RBD consisting of an amino acid sequence of amino acid residues at positions 325 to 527 of an amino acid sequence set forth in any one of SEQ ID NOs: 65, 68, 71, 74, 76, 82, 84, 87, 89, 92, 93, 94, 96, 97, 100, 102 to 104, and 106 to 110.

In the present invention, the "RBD having neutralizing antibody-inducing activity against SARS-CoV-2 and SARS-CoV-1 equivalent to that of the RBD consisting of the amino acid sequence set forth in SEQ ID NO: 1" refers to an RBD that exhibits a neutralizing antibody titer of at least 12.5% (preferably 25%, 50%, 70%, 80%, 90%, 95%, or 100% or more) relative to the neutralizing antibody titer against the SARS-CoV-2 D614G lineage (GISAID ID: EPI_ISL_529135; hereinafter also referred to as SARS-CoV-2 (D614G)) of an RBD consisting of the amino acid sequence set forth in SEQ ID NO: 1 and a neutralizing antibody titer of at least 12.5% (preferably 25%, 50%, 70%, 80%, 90%, 95%, or 100% or more) relative to the neutralizing antibody titer against SARS-CoV-1 (GenBank ID: AY278741.1) of an RBD consisting of the amino acid sequence set forth in SEQ ID NO: 1. The neutralizing antibody-inducing activity of an RBD can be determined, for example, by producing the RBD to be evaluated in accordance with the method described in Example 6, forming the RBD into nanoparticles, immunizing mice therewith, and measuring the neutralizing antibody titer of the serum obtained. The definition of the "RBD having neutralizing antibody-inducing activity against SARS-CoV-2 and SARS-CoV-1 equivalent" for sequences other than SEQ ID NO: 1 is the same as described above.

SEQ ID NO: 3 is a sequence included in H-00149 of Example 5, H-00428 of Example 6, H-00557 of Example 8, and H-00657 of Example 14, and it has been demonstrated that SEQ ID NO: 3 has neutralizing antibody-inducing activity against both SARS-CoV-2 (D614G) and SARS-CoV-1.

SEQ ID NO: 2 is a sequence included in the amino acid sequence (SEQ ID NO: 62) encoded by H-00646, H-00669, H-00672, and H-00675 of Example 14 and mRNA-001 of SEQ ID NO: 16, and it has been demonstrated that SEQ ID NO: 2 has neutralizing antibody-inducing activity against both SARS-CoV-2 (D614G) and SARS-CoV-1.

SEQ ID NO: 4 is a sequence included in H-00739 of Example 15 and SEQ ID NO: 5 is a sequence included in H-00740 of SEQ ID NO: 15, and it has been demonstrated that SEQ ID NOs: 4 and 5 are shown to have neutralizing antibody-inducing activity against both SARS-CoV-2 (D614G) and SARS-CoV-1.

As another embodiment, the spike protein variant according to the present invention comprises:
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 18, 20, 26, 29, 44, 45, 56, 65, 74, 75, and 78 in the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1; or
(c) an RBD consisting of an amino acid sequence that has substitutions, deletions, insertions, or additions at positions other than positions 18, 20, 26, 29, 44, 45, 56, 65, 74, 75, and 78 of the amino acid sequence set forth in SEQ ID NO: 1, having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1.

In the above, the "amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 18, 20, 26, 29, 44, 45, 56, 65, 74, 75, and 78 in the amino acid sequence set forth in SEQ ID NO: 1" refers to the variant sequence of the amino acid sequence set forth in SEQ ID NO: 1, in which the amino acid residues at positions 18, 20, 26, 29, 44, 45, 56, 65, 74, 75, and 78 of the amino acid sequence set forth in SEQ ID NO: 1 are not substituted, deleted, inserted, or added (i.e., the amino acid residues at positions 18, 20, 26, 29, 44, 45, 56, 65, 74, 75, and 78 are retained) and in which, at positions other than these positions, one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) are substituted, deleted, inserted, or added.

In the above, the "amino acid sequence obtained by substituting, deleting, inserting, or adding amino acids at positions other than positions 18, 20, 26, 29, 44, 45, 56, 65, 74, 75, and 78 of the amino acid sequence set forth in SEQ ID NO: 1, having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 1" refers to the variant sequence of the amino acid sequence set forth in SEQ ID NO: 1, in which the amino acid residues at positions 18, 20, 26, 29, 44, 45, 56, 65, 74, 75, and 78 are not substituted, deleted, inserted, or added(i.e., the amino acid residues at positions 18, 20, 26, 29, 44, 45, 56, 65, 74, 75, and 78 are retained) and in which, at positions other than these positions, one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) are substituted, deleted, inserted, or added, and that has, as a result of substitution, deletion, insertion, or addition at amino acid residue positions other than those described above, a sequence identity of at least 90% (preferably at least 91%, at least 92%, at least 93%, at least 94%, at least 95%, at least 96%, at least 97%, at least 98%, or at least 99%) to the amino acid sequence set forth in SEQ ID NO: 1. The same applies to SEQ ID NOs: 2 to 5.

The amino acid residues at positions 18, 20, 26, 29, 44, 45, 56, 65, 74, 75, and 78 in the amino acid sequence set forth in SEQ ID NO: 1 are considered to be important amino acid residues for exhibiting neutralizing induction activity against both SARS-CoV-2 and SARS-CoV-1, based on the experimental results of Examples 5 and 6.

As another embodiment, the spike protein variant according to the present invention comprises:
(a) an RBD consisting of an amino acid sequence set forth in SEQ ID NO: 32;
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32, or
(c) an RBD consisting of an amino acid sequence that has at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32.

As another embodiment, the spike protein variant according to the present invention comprises:
(b) an RBD consisting of an amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 44, 45, 56, 65, 74, 75, 78, 110, 111, 113, 115, 116, 117, 118, 170, 171, 173, and 175 of the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32; or
(c) an RBD consisting of an amino acid sequence obtained by substituting, deleting, inserting, or adding amino acids at positions other than positions 44, 45, 56, 65, 74, 75, 78, 110, 111, 113, 115, 116, 117, 118, 170, 171, 173, and 175 of the amino acid sequence set forth in SEQ ID NO: 32, and has at least 95% sequence identity (preferably at least 96%, at least 97%, at least 98%, or at least 99% sequence identity) to the amino acid sequence set forth in SEQ ID NO: 32, and having neutralizing antibody-inducing activity against SARS-CoV-2 and SARS-CoV-1 equivalent to that of an RBD consisting of the amino acid sequence set forth in SEQ ID NO: 32.

In the above, the "amino acid sequence obtained by substituting, deleting, inserting, or adding one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) at positions other than positions 44, 45, 56, 65, 74, 75, 78, 110, 111, 113, 115, 116, 117, 118, 170, 171, 173, and 175 of the amino acid sequence set forth in SEQ ID NO: 32" refers to the variant sequence of the amino acid sequence of SEQ ID NO: 32, in which the amino acid residues at positions 44, 45, 56, 65, 74, 75, 78, 110, 111, 113, 115, 116, 117, 118, 170, 171, 173, and 175 of the amino acid sequence set forth in SEQ ID NO: 32 are not substituted, deleted, inserted, or added(i.e., the amino acid residues at positions 44, 45, 56, 65, 74, 75, 78, 110, 111, 113, 115, 116, 117, 118, 170, 171, 173, and 175 are maintained), and in which, in amino acid residues at positions other than these positions, one to several amino acids (preferably 1 to 20, 1 to 15, 1 to 10, 1 to 9, 1 to 8, 1 to 7, 1 to 6, 1 to 5, 1 to 4, 1 to 3, or 1 to 2 amino acids) are substituted, deleted, inserted, or added.

In the above, the "amino acid sequence obtained by substituting, deleting, inserting, or adding amino acidsat positions other than positions 44, 45, 56, 65, 74, 75, 78, 110, 111, 113, 115, 116, 117, 118, 170, 171, 173, and 175 of the amino acid sequence set forth in SEQ ID NO: 32, and has at least 95% sequence identity (preferably at least 96%, at least 97%, at least 98%, or at least 99% sequence identity) to the amino acid sequence set forth in SEQ ID NO: 32" refers to a variant sequence of the amino acid sequence set forth in SEQ ID NO: 32, in which the amino acid residues at positions 44, 45, 56, 65, 74, 75, 78, 110, 111, 113, 115, 116, 117, 118, 170, 171, 173, and 175 of the amino acid sequence set forth in SEQ ID NO: 32 are not substituted, deleted, inserted, or added (i.e., the amino acid residues at positions 44, 45, 56, 65, 74, 75, 78, 110, 111, 113, 115, 116, 117, 118, 170, 171, 173, and 175 are retained), and in which as a result of substitution, deletion, insertion, or addition of the amino acid residues at the positions other than the aforementioned positions, the variant sequence has at least 90% sequence identity (preferably at least 91%, at least 92%, at least 93%, at least 94%, at least 95%, at least 96%, at least 97%, at least 98%, or at least 99%) with the amino acid sequence set forth in SEQ ID NO: 32.

The amino acid residues at positions 44, 45, 56, 65, 74, 75, 78, 110, 111, 113, 115, 116, 117, 118, 170, 171, 173, and 175 in the amino acid sequence set forth in SEQ ID NO: 32 are considered to be important amino acid residues for exhibiting neutralizing induction activity against both SARS-CoV-2 and SARS-CoV-1, based on the experimental results of Examples 5 and 6.

SEQ ID NO: 32 is a sequence included in H-000148 of Example 5, H-00081 of Example 6, and H-00556 of Example 8, and it has been demonstrated that SEQ ID NO: 32 has neutralizing antibody-inducing activity against both SARS-CoV-2 (D614G) and SARS-CoV-1.

As a further embodiment, the spike protein variant according to the present invention may further comprise:
an amino acid sequence having at least 90% sequence identity (preferably at least 91%, at least 92%, 93%, at least 94%, at least 95%, at least 96%, at least 97%, at least 98%, or at least 99% sequence identity) to amino acid residues 14 to 1211 of an amino acid sequence set forth in SEQ ID NO: 6 (full-length spike of the Delta strain).

SEQ ID NO: 6 is the full length amino acid sequence of the spike protein of the Delta strain, and the sequence is as follows.

**[Table 3]**

| Name | Amino Acid Sequence |
|---|---|
| Full Length Amino Acid Sequence of Spike Protein of Delta Strain | MFVFLVLLPLVSSQCVNLRTRTQLPPAYTNSFTRGVYYPDKVFRSSVL HSTQDLFLPFFSNVTWFHAIHVSGTNGTKRFDNPVLPFNDGVYFASTE KSNIIRGWIFGTTLDSKTQSLLIVNNATNVVIKVCEFQFCNDPFLDVY YHKNNKSWMESGVYSSANNCTFEYVSQPFLMDLEGKQGNFKNLREFVF KNIDGYFKIYSKHTPINLVRDLPQGFSALEPLVDLPIGINITRFQTLL ALHRSYLTPGDSSSGWTAGAAAYYVGYLQPRTFLLKYNENGTITDAVD CALDPLSETKCTLKSFTVEKGIYQTSNFRVQPTESIVRFPNITNLCPF GEVFNATRFASVYAWNRKRISNCVADYSVLYNSASFSTFKCYGVSPTK LNDLCFTNVYADSFVIRGDEVRQIAPGQTGKIADYNYKLPDDFTGCVI AWNSNNLDSKVGGNYNYRYRLFRKSNLKPFERDISTEIYQAGSKPCNG VEGFNCYFPLQSYGFQPTNGVGYQPYRVVVLSFELLHAPATVCGPKKS TNLVKNKCVNFNFNGLTGTGVLTESNKKFLPFQQFGRDIADTTDAVRD PQTLEILDITPCSFGGVSVITPGTNTSNQVAVLYQGVNCTEVPVAIHA DQLTPTWRVYSTGSNVFQTRAGCLIGAEHVNNSYECDIPIGAGICASY QTQTNSRRRARSVASQSIIAYTMSLGAENSVAYSNNSIAIPTNFTISV TTEILPVSMTKTSVDCTMYICGDSTECSNLLLQYGSFCTQLNRALTGI AVEQDKNTQEVFAQVKQIYKTPPIKDFGGFNFSQILPDPSKPSKRSFI EDLLFNKVTLADAGFIKQYGDCLGDIAARDLICAQKFNGLTVLPPLLT DEMIAQYTSALLAGTITSGWTFGAGAALQIPFAMQMAYRFNGIGVTQN VLYENQKLIANQFNSAIGKIQDSLSSTASALGKLQNVVNQNAQALNTL VKQLSSNFGAISSVLNDILSRLDKVEAEVQIDRLITGRLQSLQTYVTQ QLIRAAEIRASANLAATKMSECVLGQSKRVDFCGKGYHLMSFPQSAPH GVVFLHVTYVPAQEKNFTTAPAICHDGKAHFPREGVFVSNGTHWFVTQ RNFYEPQIITTDNTFVSGNCDVVIGIVNNTVYDPLQPELDSFKEELDK YFKNHTSPDVDLGDISGINASVVNIQKEIDRLNEVAKNLNESLIDLQE LGKYEQYIKWPWYIWLGFIAGLIAIVMVTIMLCCMTSCCSCLKGCCSC GSCC(SEQ ID NO: 6) |

The antigens described in the present examples (for example, H-00646, H-00657, H-00669, H-00672, H-00675, H-00739, and H-00740) include an amino acid sequence having at least 90% sequence identity to amino acids 14 to 1211 of SEQ ID NO: 6. For example, H-00657 (SEQ ID NO: 22) has an amino acid sequence having at least 96% sequence identity to amino acids 14 to 1211 of SEQ ID NO: 6.

The spike protein variant according to the present invention comprises:
an N-terminal domain (NTD) consisting of (a) an amino acid sequence set forth in SEQ ID NO: 7,
(b) an amino acid sequence obtained by substituting, deleting, inserting, or adding one to several amino acids (preferably 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 7, or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 7.

Examples of the amino acid sequence set forth in SEQ ID NO: 7 include an amino acid sequence set forth in either SEQ ID NO: 8 or SEQ ID NO: 9.

The (b) NTD consisting of an amino acid sequence obtained by substituting, deleting, inserting, or adding one to several amino acids (preferably 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 7 includes NTD consisting of an amino acid sequence consisting of amino acid residues at positions 14 to 301 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.

The N-terminal domain (NTD) refers to the N-terminal region of the coronavirus spike protein. In the ancestral strain spike protein, the amino acid sequence consisting of amino acid residues at positions 14 to 305 corresponds to NTD.

SEQ ID NOs: 8 and 9 are included in the NTD sequences of the spike protein variants according to the present invention, and SEQ ID NO: 7 is a generalized amino acid sequence of SEQ ID NOs: 8 and 9 (NTD consensus sequence). The respective amino acid sequences are as follows.

**[Table 4]**

| Name | Amino Acid Sequence |
|---|---|
| NTD Consensus Sequence | QCVNLITRTQSYTNSFTRGVYYPDKVFRSSVLHSTQDLFLPFF SNVTWFHAIHVSGTNGTKRFDNPALPFNDGVYFASTEKSNIIR GWIFGTTLDSKTQSLLIVNNATNVVIKVCEFQFCNDPFLDVYQ KNNKSWMESEFRVYSSANNCXFEYVSQPFLMDLEGKEGNFKNL REFVFKNIDGYFKIYSKHTPINLERDLPQGFSALEPLVDLPIG INITRFQTLLALHRSYLTPVDSSSGWTAGAAAYYVGYLQPRTF LLKYNENGTITDAVDCALDPLSETKCTLKS(SEQ ID NO: 7) |
| NTD Sequence 1 (H-00646,H-00657, H-00669, H-00672, H-00739, H-00740) | QCVNLITRTQSYTNSFTRGVYYPDKVFRSSVLHSTQDLFLPFF SNVTWFHAIHVSGTNGTKRFDNPALPFNDGVYFASTEKSNIIR GWIFGTTLDSKTQSLLIVNNATNVVIKVCEFQFCNDPFLDVYQ KNNKSWMESEFRVYSSANNCTFEYVSQPFLMDLEGKEGNFKNL REFVFKNIDGYFKIYSKHTPINLERDLPQGFSALEPLVDLPIG INITRFQTLLALHRSYLTPVDSSSGWTAGAAAYYVGYLQPRTF LLKYNENGTITDAVDCALDPLSETKCTLKS(SEQ ID NO: 8) |
| NTD Sequence 2 (H-00675) | QCVNLITRTQSYTNSFTRGVYYPDKVFRSSVLHSTQDLFLPFF SNVTWFHAIHVSGTNGTKRFDNPALPFNDGVYFASTEKSNIIR GWIFGTTLDSKTQSLLIVNNATNVVIKVCEFQFCNDPFLDVYQ KNNKSWMESEFRVYSSANNCLFEYVSQPFLMDLEGKEGNFKNL REFVFKNIDGYFKIYSKHTPINLERDLPQGFSALEPLVDLPIG INITRFQTLLALHRSYLTPVDSSSGWTAGAAAYYVGYLQPRTF LLKYNENGTITDAVDCALDPLSETKCTLKS(SEQ ID NO: 9) |

X at position 150 in the amino acid sequence of SEQ ID NO: 7 above is T or L.

The above-mentioned NTD sequence 1 is included in H-00646, H-00657, H-00669, H-00672, H-00739, and H-00740, and NTD sequence 2 is included in H-00675.

As another embodiment, the spike protein variant according to the present invention comprises:
an NTD consisting of (a) an amino acid sequence of a region corresponding to spike protein NTD derived from betacoronavirus (preferably from sarbecovirus),
(b) an amino acid sequence obtained by substituting, deleting, inserting, or adding one to several amino acids (preferably 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in an amino acid sequence of the region corresponding to the spike protein NTD derived from a betacoronavirus (preferably a sarbecovirus), or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence of the region corresponding to the spike protein NTD derived from betacoronavirus (preferably from sarbecovirus).

The betacoronavirus is preferably sarbecovirus. Preferred sarbecoviruses can include the following.

**[Table 5]**

| Virus Name (Lineage) | GISAID ID or GenBank ID |
|---|---|
| CH.1. | EPI_ISL_15760478 |
| BQ.1.18 | EPI_ASL_15508450 |
| XBB.1.16 | EPI_ISL_16835403 |
| XBB.1.16.1 | EPI_ISL_17030043 |
| XBF | EPI_ISL_16611536 |
| XBB.2.3 | EPI_ISL_16382405 |
| XBB.2.3.2 | EPI_ISL_17206421 |
| EG.5.1 | EPI_ISL_17471668 |
| BA.2.86 | EPI_ISL_18114953 |
| JN. 1 | EPI_ISL_18428109 |
| KP.2 | EPI_ISL_18957516 |
| KP.3 | EPI_ISL_10-59439 |
| WIV-1 | KF367457.1 |
| RaTG13 | QHR63300.2 |
| SHC014 | AGZ48806.1 |
| Pangolin-GD | WCZ55842.1 |
| Pangolin-GX | QVT76606.1 |
| HKU3 | QND76034.1 |

Examples of beta viruses other than preferred sarbecoviruses include Middle East respiratory syndrome coronavirus (MERS coronavirus, MERS-CoV).

The spike protein variant according to the present invention comprises:
a region of SD1, SD2, and S2 subunits consisting of: (a) an amino acid sequence set forth in SEQ ID NO: 10;
(b) an amino acid sequence obtained by substituting, deleting, inserting, or adding one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 10, or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 10.

Examples of the amino acid sequence set forth above in SEQ ID NO: 10 include the amino acid sequence set forth in any one of SEQ ID NOs: 11 to 15.

The region of SD1, SD2, and S2 subunits refers to the region of the coronavirus spike protein extending from subdomain 1 (SD1) and subdomain 2 (SD2) to the S2 subunit. In the case of the ancestral strain spike protein, the amino acid sequence region from position 532 to 1211 corresponds to the region of the SD1, SD2, and S2 subunits. As used herein, the region of the SD1, SD2, and S2 subunits does not include the transmembrane domain or the cytoplasmic tail. The region of the SD1, SD2 and S2 subunits may also be referred to as "SD1/2-S2".

SEQ ID NO: 10 is a sequence generalizing SEQ ID NOs: 11 to 15, which were identified as preferred sequences of the region of the SD1, SD2, and S2 subunits included in the spike protein variant according to the present invention. The respective amino acid sequences are as follows.

**[Table 6-1]**

| Name | Amino Acid Sequence |
|---|---|
| Region of SD1, SD2 and S2 Subunits (Consensus Sequence) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIXDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKXLSSNFGAISSVLNDILSRLDXPEAEVQIDRLITGRLQXL QTYVTQQLIRAAEIRASANLAATKMSECVLGQSKRVDFCGKGY HLMSFPQSAPHGVVFLHVTYVPAQEKXFTTAPAICHDGKAHFP REGVFVSXGTHWFVTQRNFYEPQIITTDNTFVSGNCDVVIGIV NNTVYDPLQPELDSFKEELDKYFKNHTSPDVDLGDISGIXASV VNIQKEIDRLNEVAKNLNESLIDLQELGKYEQYIKWP(SEQ ID NO: 10) |

**[Table 6-2]**

| Name | Amino Acid Sequence |
|---|---|
| Region 1 of SD1, SD2 and S2 Subunits (H-00646) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIKDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKQLSSNFGAISSVLNDILSRLDPPEAEVQIDRLITGRLQSL QTYVTQQLIRAAEIRASANLAATKMSECVLGQSKRVDFCGKGY HLMSFPQSAPHGVVFLHVTYVPAQEKNFTTAPAICHDGKAHFP REGVFVSNGTHWFVTQRNFYEPQIITTDNTFVSGNCDVVIGIV NNTVYDPLQPELDSFKEELDKYFKNHTSPDVDLGDISGINASV VNIQKEIDRLNEVAKNLNESLIDLQELGKYEQYIKWP(SEQ ID NO: 11) |

**[Table 7-1]**

| Name | Amino Acid Sequence |
|---|---|
| Region 2 of SD1, SD2 and S2 Subunits (H-00657) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIKDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKQLSSNFGAISSVLNDILSRLDPPEAEVQIDRLITGRLQSL QTYVTQQLIRAAEIRASANLAATKMSECVLGQSKRVDFCGKGY HLMSFPQSAPHGVVFLHVTYVPAQEKNFTTAPAICHDGKAHFP REGVFVSNGTHWFVTQRNFYEPQIITTDNTFVSGNCDVVIGIV NNTVYDPLQPELDSFKEELDKYFKNHTSPDVDLGDISGILASV VNIQKEIDRLNEVAKNLNESLIDLQELGKYEQYIKWP(SEQ ID NO: 12) |

**[Table 7-2]**

| Name | Amino Acid Sequence |
|---|---|
| Region 3 of SD1, SD2 and S2 Subunits (H-00669) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIKDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKQLSSNFGAISSVLNDILSRLDKPEAEVQIDRLITGRLQSL QTYVTQQLIRAAEIRASANLAATKMSECVLGQSKRVDFCGKGY HLMSFPQSAPHGVVFLHVTYVPAQEKNFTTAPAICHDGKAHFP REGVFVSNGTHWFVTQRNFYEPQIITTDNTFVSGNCDVVIGIV NNTVYDPLQPELDSFKEELDKYFKNHTSPDVDLGDISGINASV VNIQKEIDRLNEVAKNLNESLIDLQELGKYEQYIKWP(SEQ ID NO: 13) |

**[Table 8-1]**

| Name | Amino Acid Sequence |
|---|---|
| Region 4 of SD1, SD2 and S2 Subunits (H-00672, H-00739, H-00740) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIPDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKCLSSNFGAISSVLNDILSRLDPPEAEVQIDRLITGRLQCL QTYVTQQLIRAAEIRASANLAATKMSECVLGQSKRVDFCGKGY HLMSFPQSAPHGVVFLHVTYVPAQEKNFTTAPAICHDGKAHFP REGVFVSNGTHWFVTQRNFYEPQIITTDNTFVSGNCDVVIGIV NNTVYDPLQPELDSFKEELDKYFKNHTSPDVDLGDISGILASV VNIQKEIDRLNEVAKNLNESLIDLQELGKYEQYIKWP(SEQ ID NO: 14) |

**[Table 8-2]**

| Name | Amino Acid Sequence |
|---|---|
| Region 5 of SD1, SD2 and S2 Subunits (H-00675) | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRGSASSVASQSIIAYTMSLGAEN SVAYSNNSIAIPTNFTISVTTEILPVSMPKTSVDCTMYICGDS TECSNLLLQYGSFCTQLNRALTGIAVEQDKNTQEVFAQVKQIY KTPPIPDFGGFNFSQILPDPSKPSKRSPIEDLLFNKVTLADAG FIKQYGDCLGDIAARDLICAQKFNGLTVLPPLLTDEMIAQYTS ALLAGTITSGWTFGAGPALQIPFPMQMAYRFNGIGVTQNVLYE NQKLIANQFNSAIGKIQDSLSSTPSALGKLQNVVNQNAQALNT LVKCLSSNFGAISSVLNDILSRLDPPEAEVQIDRLITGRLQCL QTYVTQQLIRAAEIRASANLAATKMSECVLGQSKRVDFCGKGY HLMSFPQSAPHGVVFLHVTYVPAQEKQFTTAPAICHDGKAHFP REGVFVSTGTHWFVTQRNFYEPQIITTDNTFVSGNCDVVIGIV NNTVYDPLQPELDSFKEELDKYFKNHTSPDVDLGDISGILASV VNIQKEIDRLNEVAKNLNESLIDLQELGKYEQYIKWP(SEQ ID NO: 15) |

In the amino acid sequence set forth in SEQ ID NO: 10, X at position 264 is K or P, X at position 434 is Q or C, X at position 455 is P or K, X at position 472 is S or C, X at position 543 is N or Q, X at position 567 is N or T, and X at position 642 is N or L.

The region 1 of the SD1, SD2, and S2 subunits (SEQ ID NO: 11) is an amino acid sequence included in H-00646 in Example 14, the region 2 of the SD1, SD2, and S2 subunits (SEQ ID NO: 12) is an amino acid sequence included in H-00657 in Example 14, the region 3 of the SD1, SD2, and S2 subunits (SEQ ID NO: 13) is an amino acid sequence included in H-00669 in Example 14, the region 4 of the SD1, SD2, and S2 subunits (SEQ ID NO: 14) is an amino acid sequence included in H-00672, H-00739, and H-00740 in Example 14 or 15, and the region 5 of the SD1, SD2, and S2 subunits (SEQ ID NO: 15) is an amino acid sequence included in H-00675 in Example 14. These antigens show broad immunogenicity as shown in Examples 14 and 15.

The region of the SD1, SD2, and S2 subunits consisting of (b) an amino acid sequence obtained by substituting, deleting, inserting, or adding one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2 amino acids) in the amino acid sequence set forth in SEQ ID NO: 10 includes a region of SD1, SD2 and S2 subunits consisting of an amino acid sequence consisting of amino acid sequences at positions 528 to 1209 of an amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.

As another embodiment, the spike protein variant according to the present invention comprises:
a region of SD1, SD2, and S2 subunits consisting of: (a) an amino acid sequence set forth in SEQ ID NO: 16;
(b) an amino acid sequence obtained by substituting, deleting, inserting, or adding one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 16, or
(c) an amino acid sequence having at least 90% sequence identity (preferably at least 91%, at least 92%, at least 93%, at least 94%, at least 95%, at least 96%, at least 97%, at least 98%, or at least 99% sequence identity) to the amino acid sequence set forth in SEQ ID NO: 16.

SEQ ID NO: 16 corresponds to the amino acid sequence from positions 529 to 1211 of the full-length amino acid sequence (SEQ ID NO: 6) of the Delta strain spike protein, and is the following amino acid sequence.

**[Table 9]**

| Name | Amino Acid Sequence |
|---|---|
| Amino Acid Sequence of Amino-Acid Residues at Positions 529 to 1211 of Amino Acid Sequence (SEQ ID NO: 6) of Full-Length Delta Strain Spike Protein | NLVKNKCVNFNFNGLTGTGVLTESNKKFLPFQQFGRDIADTTD AVRDPQTLEILDITPCSFGGVSVITPGTNTSNQVAVLYQGVNC TEVPVAIHADQLTPTWRVYSTGSNVFQTRAGCLIGAEHVNNSY ECDIPIGAGICASYQTQTNSRRRARSVASQSIIAYTMSLGAEN SVAYSNNSIAIPTNFTISVTTEILPVSMTKTSVDCTMYICGDS TECSNLLLQYGSFCTQLNRALTGIAVEQDKNTQEVFAQVKQIY KTPPIKDFGGFNFSQILPDPSKPSKRSFIEDLLFNKVTLADAG FIKQYGDCLGDIAARDLICAQKFNGLTVLPPLLTDEMIAQYTS ALLAGTITSGWTFGAGAALQIPFAMQMAYRFNGIGVTQNVLYE NQKLIANQFNSAIGKIQDSLSSTASALGKLQNVVNQNAQALNT LVKQLSSNFGAISSVLNDILSRLDKVEAEVQIDRLITGRLQSL QTYVTQQLIRAAEIRASANLAATKMSECVLGQSKRVDFCGKGY HLMSFPQSAPHGVVFLHVTYVPAQEKNFTTAPAICHDGKAHFP REGVFVSNGTHWFVTQRNFYEPQIITTDNTFVSGNCDVVIGIV NNTVYDPLQPELDSFKEELDKYFKNHTSPDVDLGDISGINASV VNIQKEIDRLNEVAKNLNESLIDLQELGKYEQYIKWP(SEQ ID NO: 16) |

Examples of substitutions, deletions, insertions, or additions of amino acids in the "amino acid sequence in which one to several amino acids (preferably 1 to 70, 1 to 60, 1 to 50, 1 to 40, 1 to 30, 1 to 30, 1 to 25, 1 to 20, 1 to 15, 1 to 10, 1 to 9, 1 to 8, 1 to 7, 1 to 6, 1 to 5, 1 to 4, 1 to 3, or 1 to 2 amino acids) in the amino acid sequence set forth in SEQ ID NO: 16" include the following.

The spike protein variant according to the present invention may have amino acid substitution corresponding to Q434C substitution and S472C substitution in the amino acid sequence set forth in SEQ ID NO: 16. The "amino acid substitution corresponding to Q434C substitution and S472C substitution in the amino acid sequence set forth in SEQ ID NO: 16" refers to the following. That is, when the amino acid sequence of the spike protein variant of the present invention and the amino acid sequence set forth in SEQ ID NO: 16 are aligned with gaps introduced as necessary to maximize sequence identity, each amino acid residue of the spike protein variant of the present invention corresponding to Q at position 434 and S at position 472 of SEQ ID NO: 16 is substituted with C. An example of a spike protein variant having the amino acid substitutions is H-00346 in Example 12.

A spike protein variant of the present invention may comprise amino acid substitution corresponding to K455P substitution and/or V456P substitution in the amino acid sequence set forth in SEQ ID NO: 16. The "amino acid substitution corresponding to K455P substitution and/or V456P substitution in the amino acid sequence set forth in SEQ ID NO: 16" refers to, when the amino acid sequence of the spike protein variant of the present invention and the amino acid sequence set forth in SEQ ID NO: 16 are aligned with gaps introduced as necessary to maximize sequence identity, each amino acid residue of the spike protein variant of the present invention corresponding to K at position 455 and V at position 456 of SEQ ID NO: 16 is substituted with P. Examples of spike protein variants with such amino acid substitutions include H-00646, H-00657, H-00669, H-00672, H-00675, H-00739, and H-00740 in Example 14 or 15.

The spike protein variant according to the present invention may have an amino acid substitution corresponding to one or more amino acid substitutions selected from the group consisting of a F286P substitution, a A361P substitution, a A368P substitution, and a A411P substitution in the amino acid sequence set forth in SEQ ID NO: 16. The "amino acid substitution corresponding to one or more amino acid substitutions selected from the group consisting of a F286P substitution, a A361P substitution, a A368P substitution, and a A411P substitution in the amino acid sequence set forth in SEQ ID NO: 16" refers to a case in which, when the amino acid sequence of a spike protein variant according to the present invention is aligned with the amino acid sequence set forth in SEQ ID NO: 16, with gaps introduced as necessary to maximize sequence identity, one or more amino acid residues in the spike protein variant of the present invention corresponding to F at position 286, A at position 361, A at position 368, and A at position 411 of SEQ ID NO: 16 are substituted with P. The spike protein variant in which the corresponding amino acid residues are all substituted with P is preferable. Examples of the spike protein variant with such amino acid substitution(s) include H-00646, H-00657, H-00669, H-00672, H-00675, H-00739, and H-00740 of Example 14 or 15.

A spike protein variant of the present invention may comprise amino acid substitution corresponding to T201P substitution and/or K264P substitution in the amino acid sequence set forth in SEQ ID NO: 16. The "amino acid substitution corresponding to T201P substitution and/or K264P substitution in the amino acid sequence set forth in SEQ ID NO: 16" refers to, when the amino acid sequence of the spike protein variant of the present invention and the amino acid sequence set forth in SEQ ID NO: 16 are aligned with gaps introduced as necessary to maximize sequence identity, each amino acid residue of the spike protein variant of the present invention corresponding to T at position 201 and K at position 264 of SEQ ID NO: 16 is substituted with P. Examples of the above T201P substituent include H-00391 of Example 13, and examples of the K264P substituent include H-00399 of Example 12.

The spike protein variant according to the present invention may be a spike protein variant in which one or more amino acid residues corresponding to RRAR (SEQ ID NO: 17) at positions 151 to 154 of the amino acid sequence set forth in SEQ ID NO: 16 have amino acid mutations and are not cleaved by a furin protease. The "one or more amino acid residues corresponding to RRAR (SEQ ID NO: 17) at positions 151 to 154 of the amino acid sequence set forth in SEQ ID NO: 16 having amino acid mutations" refers to, when the amino acid sequence of the spike protein variant of the present invention and the amino acid sequence set forth in SEQ ID NO: 16 are aligned with gaps introduced as necessary to maximize sequence identity, amino acid mutations (substitutions, deletions, insertions, or additions) being introduced into all or one or more of amino acid residues of the spike protein variant according to the present invention corresponding to RRAR (SEQ ID NO: 17) at positions 151 to 154 of the amino acid sequence set forth in SEQ ID NO: 16. "Not cleaved by a furin protease" refers to that the intended site is not cleaved by furin protease due to the introduction of the above-mentioned amino acid mutations.

As the above-mentioned amino acid mutations, for example, the amino acid residues corresponding to RRAR (SEQ ID NO: 17) at positions 151 to 154 of the amino acid sequence set forth in SEQ ID NO: 16 may be substituted with GSAS (SEQ ID NO: 18), QQAQ (SEQ ID NO: 39), or the like. The substitution with GSAS (SEQ ID NO: 18) is preferable.

Examples of the variant having a substitution to GSAS (SEQ ID NO: 18) include H-00646, H-00657, H-00669, H-00672, H-00675, H-00739, and H-00740 in Example 14 or 15.

RRAR at positions 151 to 154 of the amino acid sequence of SEQ ID NO: 16 corresponds to the furin protease cleavage site RRAR at positions 682 to 685 in the ancestral strain spike protein, and by introducing a mutation at this site, cleavage by furin protease can be prevented, which is expected to improve stability and protein yield when expressed in cultured cells or the like.

The spike protein variant according to the present invention may have an amino acid substitution corresponding to one or more amino acid substitutions selected from the group consisting of a N543Q substitution, a N567Q substitution, and a N642L substitution in the amino acid sequence set forth in SEQ ID NO: 16. The "amino acid substitution corresponding to one or more amino acid substitutions selected from the group consisting of a N543Q substitution, a N567Q substitution, and a N643L substitution in the amino acid sequence set forth in SEQ ID NO: 16" refers to, when the amino acid sequence of the spike protein variant of the present invention and the amino acid sequence set forth in SEQ ID NO: 16 are aligned with gaps introduced as necessary to maximize sequence identity, one or more amino acid residues of the spike protein variant of the present invention corresponding to N at position 543, N at position 567, and N at position 642 of SEQ ID NO: 16 are substituted with Q, Q, or L. Examples of the spike protein variant having a N543Q substitution and a N567Q substitution include H-00511 in Example 11, and examples of the spike protein variant having a N642L substitution include H-00180 in Example 10.

The spike protein variant according to the present invention may lack the transmembrane domain and the cytoplasmic tail.

In the above, the "transmembrane domain and cytoplasmic tail" refers to the sequence corresponding to the transmembrane domain and the C-terminal cytoplasmic tail located at the C-terminus of the spike protein (positions 1211 to 1273 in the ancestral strain spike protein). By deleting this region in the spike protein, when expressed in animal cells, insect cells, or the like, the protein can be efficiently secreted extracellularly, and the efficiently expressed protein can be recovered, making it suitable as an antigen for use in vaccines.

For example, H-00646, H-00657, H-00669, H-00672, H-00675, H-00739, and H-00740 in Example 14 or 15 lack the transmembrane domain and the cytoplasmic tail.

The spike protein variant according to the present invention may include a trimerization domain, i.e., an amino acid sequence contributing to the trimer formation of the polypeptide. Preferably the trimerization domain is added to the C-terminus of the S2 subunit.

The spike protein variant according to the present invention may include a foldon sequence as a trimerization domain. Preferably, a foldon sequence is added to the C-terminus of the S2 subunit.

The foldon sequence refers to an amino acid sequence of approximately 27 amino acids, called the foldon domain, derived from the bacteriophage T4 fibritin, which contributes to trimer formation, and by expressing as a fusion protein with the foldon sequence, the protein can be stabilized.

As a preferred foldon sequence, GYIPEAPRDGQAYVRKDGEWVLLSTFL (SEQ ID NO: 19) can be exemplified, but the present invention is not limited thereto. For the foldon sequences, the foldon sequences described in WO 2008/025516 and WO 2018/081318 may be used.

Examples of H-00646, H-00657, H-00669, H-00672, H-00675, H-00739, and H-00740 in Examples 14 or 15 include GYIPEAPRDGQAYVRKDGEWVLLSTFL (SEQ ID NO: 19) as a foldon sequence.

The spike protein variant according to the present invention may include a His tag at the C-terminus. Examples of the His tag include an amino acid sequence (HHHHHH) of SEQ ID NO: 20.

By adding a His tag, the spike protein variant can be purified using a carrier containing nickel (Ni).

Examples of H-00646, H-00657, H-00669, H-00672, H-00675, H-00739, and H-00740 in Examples 14 or 15 include the amino acid sequence of SEQ ID NO: 20 (HHHHHH) at the C-terminus.

Thus, examples of spike protein variants according to the present invention also include spike protein variants comprising amino acid sequences of amino acid residues at positions 14 to C-terminus of the amino acid sequence set forth in any one of SEQ ID NOs: 21 to 27. The amino acid sequence (HHHHHH) at the C-terminus is used for protein purification and is not an essential element for use as an antigen.

Thus, examples of spike protein variants according to the present invention also include spike protein variants comprising amino acid sequences of amino acid residues at positions 14 to 1236 of the amino acid sequences set forth in any of SEQ ID NOs: 21 to 27. In addition, since the spike protein variants according to the present invention may lack a trimerization domain, examples of the spike protein variants according to the present invention also include spike protein variants including amino acid residues 14 to 1209 of the amino acid sequence set forth in any one of SEQ ID NOs: 21 to 27.

The amino acid sequences set forth in any one of SEQ ID NOs: 21 to 27 are shown below.

**[Table 10]**

| Name | Amino Acid Sequence |
|---|---|
| H-00646 | MFVFLVLLPLVSSQCVNLITRTQSYTNSFTRGVYYPDKVFRSSVLHS TQDLFLPFFSNVTWFHAIHVSGTNGTKRFDNPALPFNDGVYFASTEK SNIIRGWIFGTTLDSKTQSLLIVNNATNVVIKVCEFQFCNDPFLDVY QKNNKSWMESEFRVYSSANNCTFEYVSQPFLMDLEGKEGNFKNLREF VFKNIDGYFKIYSKHTPINLERDLPQGFSALEPLVDLPIGINITRFQ TLLALHRSYLTPVDSSSGWTAGAAAYYVGYLQPRTFLLKYNENGTIT DAVDCALDPLSETKCTLKSFTVEKGIYQTSNFRVQPTESIVRFPQIT NLCPFGEVFNATKFPSVYAWERKKISNCVADYSVLYNSTFFSTFKCY GVSATKLNDLCFSNVYADSFVVKGDDVRQIAPGQTGVIADYNYKLPD DFMGCVIAWNSNNLDSKVGGNYNYRYRLFRKSNLKPFERDISTEIYQ AGSKPCNGVEGFNCYFPLQSYGFQPTNGVGYQPYRVVVLSFELLHAP ATVCGPKKSTNLVKNKCVNFNFNGLTGTGVLTESNKKFLPFQQFGRD IADTTDAVRDPQTLEILDITPCSFGGVSVITPGTNTSNQVAVLYQGV NCTEVPVAIHADQLTPTWRVYSTGSNVFQTRAGCLIGAEHVNNSYEC DIPIGAGICASYQTQTNSRGSASSVASQSIIAYTMSLGAENSVAYSN NSIAIPTNFTISVTTEILPVSMPKTSVDCTMYICGDSTECSNLLLQY GSFCTQLNRALTGIAVEQDKNTQEVFAQVKQIYKTPPIKDFGGFNFS QILPDPSKPSKRSPIEDLLFNKVTLADAGFIKQYGDCLGDIAARDLI CAQKFNGLTVLPPLLTDEMIAQYTSALLAGTITSGWTFGAGPALQIP FPMQMAYRFNGIGVTQNVLYENQKLIANQFNSAIGKIQDSLSSTPSA LGKLQNVVNQNAQALNTLVKQLSSNFGAISSVLNDILSRLDPPEAEV QIDRLITGRLQSLQTYVTQQLIRAAEIRASANLAATKMSECVLGQSK RVDFCGKGYHLMSFPQSAPHGVVFLHVTYVPAQEKNFTTAPAICHDG KAHFPREGVFVSNGTHWFVTQRNFYEPQIITTDNTFVSGNCDVVIGI VNNTVYDPLQPELDSFKEELDKYFKNHTSPDVDLGDISGINASVVNI QKEIDRLNEVAKNLNESLIDLQELGKYEQYIKWPGYIPEAPRDGQAY VRKDGEWVLLSTFLHHHHHH(SEQ ID NO: 21) |

**[Table 11]**

| Name | Amino Acid Sequence |
|---|---|
| H-00657 | MFVFLVLLPLVSSQCVNLITRTQSYTNSFTRGVYYPDKVFRSSVLH STQDLFLPFFSNVTWFHAIHVSGTNGTKRFDNPALPFNDGVYFAST EKSNIIRGWIFGTTLDSKTQSLLIVNNATNVVIKVCEFQFCNDPFL DVYQKNNKSWMESEFRVYSSANNCTFEYVSQPFLMDLEGKEGNFKN LREFVFKNIDGYFKIYSKHTPINLERDLPQGFSALEPLVDLPIGIN ITRFQTLLALHRSYLTPVDSSSGWTAGAAAYYVGYLQPRTFLLKYN ENGTITDAVDCALDPLSETKCTLKSFTVEKGIYQTSNFRVQPTESI VRFPNITNLCPFGEVFNATKFPSVYAWERKKISNCVADYSVLYNST FFSTFKCYGVSATKLNDLCFSNVYADSFVVKGDDVRQIAPGQTGVI ADYNYKLPDDFMGCVIAWNSNNLDSKVGGNYNYRYRLFRKSNLKPF ERDISTEIYQAGSKPCNGVEGFNCYFPLQSYGFQPTNGVGYQPYRV VVLSFELLHAPATVCGPKKSTNLVKNKCVNFNFNGLTGTGVLTESN KKFLPFQQFGRDIADTTDAVRDPQTLEILDITPCSFGGVSVITPGT NTSNQVAVLYQGVNCTEVPVAIHADQLTPTWRVYSTGSNVFQTRAG CLIGAEHVNNSYECDIPIGAGICASYQTQTNSRGSASSVASQSIIA YTMSLGAENSVAYSNNSIAIPTNFTISVTTEILPVSMPKTSVDCTM YICGDSTECSNLLLQYGSFCTQLNRALTGIAVEQDKNTQEVFAQVK QIYKTPPIKDFGGFNFSQILPDPSKPSKRSPIEDLLFNKVTLADAG FIKQYGDCLGDIAARDLICAQKFNGLTVLPPLLTDEMIAQYTSALL AGTITSGWTFGAGPALQIPFPMQMAYRFNGIGVTQNVLYENQKLIA NQFNSAIGKIQDSLSSTPSALGKLQNVVNQNAQALNTLVKQLSSNF GAISSVLNDILSRLDPPEAEVQIDRLITGRLQSLQTYVTQQLIRAA EIRASANLAATKMSECVLGQSKRVDFCGKGYHLMSFPQSAPHGVVF LHVTYVPAQEKNFTTAPAICHDGKAHFPREGVFVSNGTHWFVTQRN FYEPQIITTDNTFVSGNCDVVIGIVNNTVYDPLQPELDSFKEELDK YFKNHTSPDVDLGDISGILASVVNIQKEIDRLNEVAKNLNESLIDL QELGKYEQYIKWPGYIPEAPRDGQAYVRKDGEWVLLSTFLHHHHHH (SEQ ID NO: 22) |

**[Table 12]**

| Name | Amino Acid Sequence |
|---|---|
| H-00669 | MFVFLVLLPLVSSQCVNLITRTQSYTNSFTRGVYYPDKVFRSSVLHS TQDLFLPFFSNVTWFHAIHVSGTNGTKRFDNPALPFNDGVYFASTEK SNIIRGWIFGTTLDSKTQSLLIVNNATNVVIKVCEFQFCNDPFLDVY QKNNKSWMESEFRVYSSANNCTFEYVSQPFLMDLEGKEGNFKNLREF VFKNIDGYFKIYSKHTPINLERDLPQGFSALEPLVDLPIGINITRFQ TLLALHRSYLTPVDSSSGWTAGAAAYYVGYLQPRTFLLKYNENGTIT DAVDCALDPLSETKCTLKSFTVEKGIYQTSNFRVQPTESIVRFPQIT NLCPFGEVFNATKFPSVYAWERKKISNCVADYSVLYNSTFFSTFKCY GVSATKLNDLCFSNVYADSFVVKGDDVRQIAPGQTGVIADYNYKLPD DFMGCVIAWNSNNLDSKVGGNYNYRYRLFRKSNLKPFERDISTEIYQ AGSKPCNGVEGFNCYFPLQSYGFQPTNGVGYQPYRVVVLSFELLHAP ATVCGPKKSTNLVKNKCVNFNFNGLTGTGVLTESNKKFLPFQQFGRD IADTTDAVRDPQTLEILDITPCSFGGVSVITPGTNTSNQVAVLYQGV NCTEVPVAIHADQLTPTWRVYSTGSNVFQTRAGCLIGAEHVNNSYEC DIPIGAGICASYQTQTNSRGSASSVASQSIIAYTMSLGAENSVAYSN NSIAIPTNFTISVTTEILPVSMPKTSVDCTMYICGDSTECSNLLLQY GSFCTQLNRALTGIAVEQDKNTQEVFAQVKQIYKTPPIKDFGGFNFS QILPDPSKPSKRSPIEDLLFNKVTLADAGFIKQYGDCLGDIAARDLI CAQKFNGLTVLPPLLTDEMIAQYTSALLAGTITSGWTFGAGPALQIP FPMQMAYRFNGIGVTQNVLYENQKLIANQFNSAIGKIQDSLSSTPSA LGKLQNVVNQNAQALNTLVKQLSSNFGAISSVLNDILSRLDKPEAEV QIDRLITGRLQSLQTYVTQQLIRAAEIRASANLAATKMSECVLGQSK RVDFCGKGYHLMSFPQSAPHGVVFLHVTYVPAQEKNFTTAPAICHDG KAHFPREGVFVSNGTHWFVTQRNFYEPQIITTDNTFVSGNCDVVIGI VNNTVYDPLQPELDSFKEELDKYFKNHTSPDVDLGDISGINASVVNI QKEIDRLNEVAKNLNESLIDLQELGKYEQYIKWPGYIPEAPRDGQAY VRKDGEWVLLSTFLHHHHHH(SEQ ID NO: 23) |

**[Table 13]**

| Name | Amino Acid Sequence |
|---|---|
| H-00672 | MFVFLVLLPLVSSQCVNLITRTQSYTNSFTRGVYYPDKVFRSSVLH STQDLFLPFFSNVTWFHAIHVSGTNGTKRFDNPALPFNDGVYFAST EKSNIIRGWIFGTTLDSKTQSLLIVNNATNVVIKVCEFQFCNDPFL DVYQKNNKSWMESEFRVYSSANNCTFEYVSQPFLMDLEGKEGNFKN LREFVFKNIDGYFKIYSKHTPINLERDLPQGFSALEPLVDLPIGIN ITRFQTLLALHRSYLTPVDSSSGWTAGAAAYYVGYLQPRTFLLKYN ENGTITDAVDCALDPLSETKCTLKSFTVEKGIYQTSNFRVQPTESI VRFPQITNLCPFGEVFNATKFPSVYAWERKKISNCVADYSVLYNST FFSTFKCYGVSATKLNDLCFSNVYADSFVVKGDDVRQIAPGQTGVI ADYNYKLPDDFMGCVIAWNSNNLDSKVGGNYNYRYRLFRKSNLKPF ERDISTEIYQAGSKPCNGVEGFNCYFPLQSYGFQPTNGVGYQPYRV VVLSFELLHAPATVCGPKKSTNLVKNKCVNFNFNGLTGTGVLTESN KKFLPFQQFGRDIADTTDAVRDPQTLEILDITPCSFGGVSVITPGT NTSNQVAVLYQGVNCTEVPVAIHADQLTPTWRVYSTGSNVFQTRAG CLIGAEHVNNSYECDIPIGAGICASYQTQTNSRGSASSVASQSIIA YTMSLGAENSVAYSNNSIAIPTNFTISVTTEILPVSMPKTSVDCTM YICGDSTECSNLLLQYGSFCTQLNRALTGIAVEQDKNTQEVFAQVK QIYKTPPIPDFGGFNFSQILPDPSKPSKRSPIEDLLFNKVTLADAG FIKQYGDCLGDIAARDLICAQKFNGLTVLPPLLTDEMIAQYTSALL AGTITSGWTFGAGPALQIPFPMQMAYRFNGIGVTQNVLYENQKLIA NQFNSAIGKIQDSLSSTPSALGKLQNVVNQNAQALNTLVKCLSSNF GAISSVLNDILSRLDPPEAEVQIDRLITGRLQCLQTYVTQQLIRAA EIRASANLAATKMSECVLGQSKRVDFCGKGYHLMSFPQSAPHGVVF LHVTYVPAQEKNFTTAPAICHDGKAHFPREGVFVSNGTHWFVTQRN FYEPQIITTDNTFVSGNCDVVIGIVNNTVYDPLQPELDSFKEELDK YFKNHTSPDVDLGDISGILASVVNIQKEIDRLNEVAKNLNESLIDL QELGKYEQYIKWPGYIPEAPRDGQAYVRKDGEWVLLSTFLHHHHHH (SEQ ID NO: 24) |

**[Table 14]**

| Name | Amino Acid Sequence |
|---|---|
| H-00675 | MFVFLVLLPLVSSQCVNLITRTQSYTNSFTRGVYYPDKVFRSSVLH STQDLFLPFFSNVTWFHAIHVSGTNGTKRFDNPALPFNDGVYFAST EKSNIIRGWIFGTTLDSKTQSLLIVNNATNVVIKVCEFQFCNDPFL DVYQKNNKSWMESEFRVYSSANNCLFEYVSQPFLMDLEGKEGNFKN LREFVFKNIDGYFKIYSKHTPINLERDLPQGFSALEPLVDLPIGIN ITRFQTLLALHRSYLTPVDSSSGWTAGAAAYYVGYLQPRTFLLKYN ENGTITDAVDCALDPLSETKCTLKSFTVEKGIYQTSNFRVQPTESI VRFPQITNLCPFGEVFNATKFPSVYAWERKKISNCVADYSVLYNST FFSTFKCYGVSATKLNDLCFSNVYADSFVVKGDDVRQIAPGQTGVI ADYNYKLPDDFMGCVIAWNSNNLDSKVGGNYNYRYRLFRKSNLKPF ERDISTEIYQAGSKPCNGVEGFNCYFPLQSYGFQPTNGVGYQPYRV VVLSFELLHAPATVCGPKKSTNLVKNKCVNFNFNGLTGTGVLTESN KKFLPFQQFGRDIADTTDAVRDPQTLEILDITPCSFGGVSVITPGT NTSNQVAVLYQGVNCTEVPVAIHADQLTPTWRVYSTGSNVFQTRAG CLIGAEHVNNSYECDIPIGAGICASYQTQTNSRGSASSVASQSIIA YTMSLGAENSVAYSNNSIAIPTNFTISVTTEILPVSMPKTSVDCTM YICGDSTECSNLLLQYGSFCTQLNRALTGIAVEQDKNTQEVFAQVK QIYKTPPIPDFGGFNFSQILPDPSKPSKRSPIEDLLFNKVTLADAG FIKQYGDCLGDIAARDLICAQKFNGLTVLPPLLTDEMIAQYTSALL AGTITSGWTFGAGPALQIPFPMQMAYRFNGIGVTQNVLYENQKLIA NQFNSAIGKIQDSLSSTPSALGKLQNVVNQNAQALNTLVKCLSSNF GAISSVLNDILSRLDPPEAEVQIDRLITGRLQCLQTYVTQQLIRAA EIRASANLAATKMSECVLGQSKRVDFCGKGYHLMSFPQSAPHGVVF LHVTYVPAQEKQFTTAPAICHDGKAHFPREGVFVSTGTHWFVTQRN FYEPQIITTDNTFVSGNCDVVIGIVNNTVYDPLQPELDSFKEELDK YFKNHTSPDVDLGDISGILASVVNIQKEIDRLNEVAKNLNESLIDL QELGKYEQYIKWPGYIPEAPRDGQAYVRKDGEWVLLSTFLHHHHHH (SEQ ID NO: 25) |

**[Table 15]**

| Name | Amino Acid Sequence |
|---|---|
| H-00739 | MFVFLVLLPLVSSQCVNLITRTQSYTNSFTRGVYYPDKVFRSSVLH STQDLFLPFFSNVTWFHAIHVSGTNGTKRFDNPALPFNDGVYFAST EKSNIIRGWIFGTTLDSKTQSLLIVNNATNVVIKVCEFQFCNDPFL DVYQKNNKSWMESEFRVYSSANNCTFEYVSQPFLMDLEGKEGNFKN LREFVFKNIDGYFKIYSKHTPINLERDLPQGFSALEPLVDLPIGIN ITRFQTLLALHRSYLTPVDSSSGWTAGAAAYYVGYLQPRTFLLKYN ENGTITDAVDCALDPLSETKCTLKSFTVEKGIYQTSNFRVQPTESI VRFPQITNLCPFGEVFNATKFPSVYAWERKKISNCVADYSVLYNST FFSTFKCYGVSATKLNDLCFSNVYADSFVVKGDDVRQIAPGQTGVI ADYNYKLPDDFMGCVIAWNSNNLDSKVGGNYNYRYRLFRKSKLKPF ERDISTEIYQAGSKPCNGVEGFNCYFPLQSYGFQPTYGVGYQPYRV VVLSFELLHAPATVCGPKKSTNLVKNKCVNFNFNGLTGTGVLTESN KKFLPFQQFGRDIADTTDAVRDPQTLEILDITPCSFGGVSVITPGT NTSNQVAVLYQGVNCTEVPVAIHADQLTPTWRVYSTGSNVFQTRAG CLIGAEHVNNSYECDIPIGAGICASYQTQTNSRGSASSVASQSIIA YTMSLGAENSVAYSNNSIAIPTNFTISVTTEILPVSMPKTSVDCTM YICGDSTECSNLLLQYGSFCTQLNRALTGIAVEQDKNTQEVFAQVK QIYKTPPIPDFGGFNFSQILPDPSKPSKRSPIEDLLFNKVTLADAG FIKQYGDCLGDIAARDLICAQKFNGLTVLPPLLTDEMIAQYTSALL AGTITSGWTFGAGPALQIPFPMQMAYRFNGIGVTQNVLYENQKLIA NQFNSAIGKIQDSLSSTPSALGKLQNVVNQNAQALNTLVKCLSSNF GAISSVLNDILSRLDPPEAEVQIDRLITGRLQCLQTYVTQQLIRAA EIRASANLAATKMSECVLGQSKRVDFCGKGYHLMSFPQSAPHGVVF LHVTYVPAQEKNFTTAPAICHDGKAHFPREGVFVSNGTHWFVTQRN FYEPQIITTDNTFVSGNCDVVIGIVNNTVYDPLQPELDSFKEELDK YFKNHTSPDVDLGDISGILASVVNIQKEIDRLNEVAKNLNESLIDL QELGKYEQYIKWPGYIPEAPRDGQAYVRKDGEWVLLSTFLHHHHHH (SEQ ID NO: 26) |

**[Table 16]**

| Name | Amino Acid Sequence |
|---|---|
| H-00740 | MFVFLVLLPLVSSQCVNLITRTQSYTNSFTRGVYYPDKVFRSSVLH STQDLFLPFFSNVTWFHAIHVSGTNGTKRFDNPALPFNDGVYFAST EKSNIIRGWIFGTTLDSKTQSLLIVNNATNVVIKVCEFQFCNDPFL DVYQKNNKSWMESEFRVYSSANNCTFEYVSQPFLMDLEGKEGNFKN LREFVFKNIDGYFKIYSKHTPINLERDLPQGFSALEPLVDLPIGIN ITRFQTLLALHRSYLTPVDSSSGWTAGAAAYYVGYLQPRTFLLKYN ENGTITDAVDCALDPLSETKCTLKSFTVEKGIYQTSNFRVQPTESI VRFPQITNLCPFGEVFNATKFPSVYAWERKKISNCVADYSVLYNST FFSTFKCYGVSATKLNDLCFSNVYADSFVVKGDDVRQIAPGQTGVI ADYNYKLPDDFMGCVIAWNSNNLDSKVGGNYNYRYRLFRKSKLKPF ERDISTEIYQAGNKPCNGVEGFNCYFPLQSYGFQPTYGVGYQPYRV VVLSFELLHAPATVCGPKKSTNLVKNKCVNFNFNGLTGTGVLTESN KKFLPFQQFGRDIADTTDAVRDPQTLEILDITPCSFGGVSVITPGT NTSNQVAVLYQGVNCTEVPVAIHADQLTPTWRVYSTGSNVFQTRAG CLIGAEHVNNSYECDIPIGAGICASYQTQTNSRGSASSVASQSIIA YTMSLGAENSVAYSNNSIAIPTNFTISVTTEILPVSMPKTSVDCTM YICGDSTECSNLLLQYGSFCTQLNRALTGIAVEQDKNTQEVFAQVK QIYKTPPIPDFGGFNFSQILPDPSKPSKRSPIEDLLFNKVTLADAG FIKQYGDCLGDIAARDLICAQKFNGLTVLPPLLTDEMIAQYTSALL AGTITSGWTFGAGPALQIPFPMQMAYRFNGIGVTQNVLYENQKLIA NQFNSAIGKIQDSLSSTPSALGKLQNVVNQNAQALNTLVKCLSSNF GAISSVLNDILSRLDPPEAEVQIDRLITGRLQCLQTYVTQQLIRAA EIRASANLAATKMSECVLGQSKRVDFCGKGYHLMSFPQSAPHGVVF LHVTYVPAQEKNFTTAPAICHDGKAHFPREGVFVSNGTHWFVTQRN FYEPQIITTDNTFVSGNCDVVIGIVNNTVYDPLQPELDSFKEELDK YFKNHTSPDVDLGDISGILASVVNIQKEIDRLNEVAKNLNESLIDL QELGKYEQYIKWPGYIPEAPRDGQAYVRKDGEWVLLSTFLHHHHHH (SEQ ID NO: 27) |

Amino-acid residues at positions 1 to 13 on the N-terminus side of the amino acid sequence listed in any one of SEQ ID NOs: 21 to 27 constitute a secretory signal sequence, which is required for extracellular secretion when expressed in mammalian or insect-derived cultured cells.

Thus, examples of spike protein variants according to the present invention also include spike protein variants comprising amino acid sequences of amino acid residues at positions 14 to C-terminus of the amino acid sequence set forth in any one of SEQ ID NOs: 65 to 122. The amino acid sequence (HHHHHH) at the C-terminus is used for protein purification and is not an essential element for use as an antigen.

Thus, examples of spike protein variants according to the present invention also include spike protein variants comprising amino acid sequences of amino acid residues at positions 14 to 1236 of the amino acid sequences set forth in any of SEQ ID NOs: 65 to 122. In addition, since the spike protein variants according to the present invention may lack a trimerization domain, examples of the spike protein variants according to the present invention also include spike protein variants including amino acid residues 14 to 1209 of the amino acid sequence set forth in any one of SEQ ID NOs: 65 to 122.

The amino acid sequences set forth in any one of SEQ ID NOs: 65 to 122 are shown below.

**[Table 17]**

| Name | SEQ ID NO. | Name | SEQ ID NO. |
|---|---|---|---|
| H-00885 | 65 | H-00987 | 94 |
| H-00954 | 66 | H-00980 | 95 |
| H-00901 | 67 | H-00895 | 96 |
| H-00881 | 68 | H-00870 | 97 |
| H-01059 | 69 | H-00967 | 98 |
| H-00952 | 70 | H-00905 | 99 |
| H-00986 | 71 | H-00886 | 100 |
| H-00945 | 72 | H-00951 | 101 |
| H-01063 | 73 | H-00890 | 102 |
| H-00854 | 74 | H-00920 | 103 |
| H-00953 | 75 | H-00862 | 104 |
| H-00874 | 76 | H-00893 | 105 |
| H-00962 | 77 | H-00891 | 106 |
| H-00807 | 78 | H-00889 | 107 |
| H-00783 | 79 | H-00872 | 108 |
| H-00848 | 80 | H-00934 | 109 |
| H-00990 | 81 | H-00892 | 110 |
| H-00916 | 82 | H-00956 | 111 |
| H-00794 | 83 | H-01017 | 112 |
| H-00914 | 84 | H-01024 | 113 |
| H-00958 | 85 | H-01030 | 114 |
| H-00909 | 86 | H-01036 | 115 |
| H-00917 | 87 | H-01039 | 116 |
| H-00955 | 88 | H-01044 | 117 |
| H-00915 | 89 | H-01046 | 118 |
| H-00997 | 90 | H-01047 | 119 |
| H-00789 | 91 | H-01048 | 120 |
| H-00829 | 92 | H-01049 | 121 |
| H-00976 | 93 | H-01112 | 122 |

The immunogenic composition according to the present invention comprises the spike protein variant according to the present invention.

Preferably, the immunogenic composition according to the present invention comprises only one type of spike protein variant according to the present invention as an antigen derived from a coronavirus spike protein, and not comprising any other antigen derived from the coronavirus spike protein. That is, the spike protein variant according to the present invention is broadly immunogenic by monovalent antigens, and are effective as broadly directed vaccines.

The immunogenic composition according to the present invention can be used for the prevention of infections caused by SARS-CoV-2, and for the prevention of infections caused by other coronaviruses (preferably betacoronavirus, more preferably sarbecovirus).

The immunogenic composition according to the present invention may include an adjuvant.

Preferred adjuvant is an adjuvant comprising squalene, tocopherol, and polysorbate 80. For example, A-910823, a squalene-based adjuvant, can be specifically exemplified. Details of A-910823 are described, for example, in Front Immunol. 2023 Feb 20;14:1116238 (Reference Document 1).

As preferred adjuvants, an adjuvant obtained by mixing an aluminum salt, Alum, with an oligodeoxynucleotide, ODN2006, or an adjuvant obtained by mixing an aluminum salt, Alum, with an oligodeoxynucleotide, CpG1018, can be exemplified.

The present invention includes nucleic acids encoding the spike protein variants according to the present invention. Specifically, the coronavirus spike protein variant according to the present invention may be encoded in DNA plasmid expression vectors or mRNA, and expressed in vivo. The spike protein variant according to the present invention encoded by the nucleic acid encoding the spike protein variant according to the present invention may, but need not, include a C-terminal trimerization domain and/or a His tag, and may include a transmembrane domain and a cytoplasmic tail.

The present invention includes an expression vector including a nucleic acid encoding the spike protein variant according to the present invention. The expression vector may include a virus vector (e.g., an adenovirus vector).

The coronavirus spike protein variant according to the present invention can be produced using known gene-engineering techniques. For example, the coronavirus spike protein variant may be expressed using host cells such as mammalian-derived cultured cells or insect-derived cultured cells, and methods well known to those skilled in the art may be employed. The coronavirus spike protein variant according to the present invention may also be encoded in a plasmid expression vector, a virus vector (e.g., adenovirus vector), or mRNA, and expressed in vivo.

The dosage form of the immunogenic composition can be appropriately selected. The composition may be in the form of a liquid formulation, a powder formulation, or a lyophilized formulation.

The route of administration of the immunogenic composition of the present invention can be appropriately selected and may be oral or non-oral. Examples of the non-oral administration include, but are not limited to, intranasal administration, inhalation, nasal cavity administration, intraperitoneal administration, intramuscular administration, transdermal administration, subcutaneous administration, intradermal administration, sublingual administration, intravenous administration, enteral administration, and transmucosal administration.

The immunogenic composition of the present invention may, depending on the purpose or application, optionally contain additives such as buffers, tonicity agents, anesthetics, preservatives, antimicrobial agents, antioxidants, pH adjusters, dispersing agents, fragrances, colorants, and antifoaming agents.

The dosage of the immunogenic composition according to the present invention can be appropriately selected based on the type of active ingredient, the route of administration, the subject to be administered, the age, weight, and sex of the patient, symptoms, and other relevant conditions.

Hereinafter, the present invention will be described in more detail by way of Examples; however, the present invention is not intended to be limited by these Examples.

### [Example 1]

### Method of Producing Coronavirus Spike Protein Variants Using Expi293F Cells

Expi293F cells were used as the production system for the coronavirus spike protein variant. An expression vector encoding the spike protein variant (a mammalian expression plasmid including a cytomegalovirus an immediate early enhancer-chicken β-actin hybrid (CAG) promoter, a woodchuck hepatitis virus post-transcriptional regulatory element (WPRE) sequence, and a rabbit β-globin polyadenylation signal sequence) was transfected into Expi293F cells using the ExpiFectamine^{™} 293 Transfection Kit (Thermo Fisher Scientific). The experiment was generally performed according to the protocol provided with the Kit, but Kifunenesine (final concentration of 10 µM) was added simultaneously during transfection. One day after the transfection, Enhancer 1 and Enhancer 2 provided with the kit were mixed immediately before use and added to the cells. Four to five days after transfection, the culture supernatant was collected, and the spike protein variant was obtained from the supernatant.

The culture supernatants containing the respective spike protein variants obtained above were purified using Talon (registered trademark) Metal Affinity Resin (Takara Bio Inc.) or NGL COVID-19 Spike Protein Affinity Resin (REPLIGEN), thereby obtaining purified spike protein variants. The purified antigen proteins were concentrated and buffer-exchanged into PBS buffer (GIBCO) using an Amicon (registered trademark) Ultra-2 Centrifugal Filter Unit 100 kDa NWMCO (Merck Millipore). The purity of the obtained purified spike protein variants was confirmed by gel electrophoresis and CBB staining. In addition, the concentration of the purified spike proteins was determined by a protein quantification assay using a bicinchoninic acid (BCA) reagent.

### [Example 2]

### Method of Nanoparticulating RBD-only Antigens

Each RBD protein with a 6×His-Avi-tag was transfected in the same manner as in Example 1, and after transfection, the cells were cultured in the presence of 100 µM biotin. The culture supernatants containing the respective RBD protein variants were purified using Talon Metal Affinity Resin (Takara Bio Inc.) to obtain biotinylated RBD proteins. The biotinylated RBD was mixed with streptavidin-coated nanoparticles (Bangs Laboratories) to prepare biotinylated RBD-bound nanoparticles. The excess amount of biotinylated RBD and non-biotinylated RBD that did not bind to the nanoparticles were removed using a Nanosep centrifugal ultrafiltration device 300 kDa NWMCO (Pall Japan), and the buffer was exchanged into PBS buffer (GIBCO).

### [Example 3]

### Method of Producing Coronavirus Spike Protein Variant Using Baculovirus Expression System (BEVS)

As hosts for producing coronavirus spike protein variants, insect cells such as Spodoptera frugiperda (Sf) cells (e.g., Sf9 cells) and Trichoplusia ni (Tn) cells (e.g., High Five cells) were used. A baculovirus vector encoding a spike protein variant (e.g., pVL1392) and a baculovirus genome (e.g., flashBAC from Oxford Expression Technologies) were co-transfected, and homologous recombination was allowed to occur in insect cells to generate a baculovirus. Cells were cultured in a bioreactor, spinner flask, or the like, and infected with a baculovirus to express the respective spike protein variants. Three days after infection, the cells or the culture supernatant was collected. Full-length spike proteins including a transmembrane domain were recovered from host cells by extraction using a surfactant. The ectodomain, which is an extracellular domain, could be recovered from the culture supernatant, and was concentrated and buffer-exchanged by tangential flow filtration (TFF). For each of the full-length spike protein and the ectodomain, a first purification step was performed using a His-tag affinity resin or a spike protein receptor-binding domain (RBD) affinity resin (e.g., NGL COVID-19 Spike Protein Affinity Resin from Repligen). As a second purification step, purification was performed using a hydrophobic interaction resin or an ion-exchange resin. After nucleic acids were removed using an anion-exchange membrane, the product was concentrated and buffer-exchanged using a TFF system or a combination of ultrafiltration and dialysis, and then passed through a 0.22 µm filter to obtain an antigen drug substance. The obtained antigen drug substance was assayed for protein content by the bicinchoninic acid (BCA) method, and purity was evaluated by staining the proteins separated by SDS-PAGE.

### [Example 4]

### Neutralizing Activity Assay for Variant Spike Protein

### (Efficacy Test Method)

### (1) Mouse Immunization

The spike protein derived from Expi293F cells prepared by the method of Example 1 or 2 or the spike protein derived from BEVS (Baculovirus Expression Vector System) prepared by the method of Example 3 was mixed 1:1 with a squalene-based emulsion adjuvant, A-910823 (Reference Document 1), to prepare an antigen solution at 20 µg/ml. Fifty microliters of the solution was administered intramuscularly to female BALB/c mice at 1 µg/mouse (n = 5 per group). At 2 weeks after the initial administration, the same immunogen was administered in the same manner as described above.

For the mRNA antigens described in Example 16, 50 µl was administered intramuscularly in female BALB/c mice to be 0.3 µg/mouse or 3 µg/mouse without adjuvant (5 per group). At 3 weeks after the initial administration, the same immunogen was administered in the same manner as described above.

### (2) Serum Collection

At 2 weeks after the final administration, blood was collected from the inferior vena cava of the mice under isoflurane anesthesia, and serum was separated using a serum separation tube containing a clot activator. The serum was inactivated by heating at 56°C for 30 minutes.

### (3) Neutralizing Activity Assay

A pseudovirus neutralization assay was performed using the heat-inactivated sera obtained in (2) above. Specifically, a solution prepared by mixing serially diluted serum with pseudovirus was added to 293T-AT cells seeded in 96- or 384-well plates, and the cells were cultured for 2 days at 37°C in the presence of 5% carbon dioxide. Thereafter, ONE-Glo Reagent was added to the cells, and luminescence was measured using a plate reader. The neutralizing antibody titer (NT50) is the serum dilution factor that inhibits infection by 50% in the pseudovirus assay. The neutralizing antibody titer (NT50) was calculated using XLfit. If the inhibitory rate % at the lowest dilution was below 50%, the NT50 value was set to one-half of the lowest dilution factor. If the inhibitory rate % at the highest dilution was above 50%, the NT50 value was set to the highest dilution factor. The graphs in Figs. 1 to 24 show the neutralizing antibody titers for individual animals and the geometric mean values with 95% confidence intervals for each group. The method for producing the pseudovirus is described in Vaccine. 2022 Jul 29; 40(31): 4231-4241 (Reference Document 2). The accession numbers of the spike proteins of the respective viral strains used for the pseudovirus are shown in the table below.

**[Table 18]**

| Virus Name (Lineage) | GISAID ID or GenBank ID |
|---|---|
| D614G | EPI_ISL_529135 |
| Beta | EPI_ISL_768642 |
| Delta | EPI_ISL_1914591 |
| BA.1 | EPI_ISL_6640917 |
| BA.2 | EPI_ISL_9595859 |
| BA.4/5 | EPI_ISL_11542270 |
| BA.2.75 | EPI _ISL_13409465 |
| BQ.1.1 | EPI_ISL_15115256 |
| XBB.1 | EPI_ISL_14917761 |
| XBB.1.5 | EPI_ISL_15706596 |
| SARS-1 | AY278741.1 |
| WIV-1 | KF367457.1 |

### [Example 5]

### Neutralizing Antibody Induction Assay of Spike Protein Carrying RBD Chimera

To design an RBD sequence capable of conferring neutralizing activity against both SARS-CoV-1 and SARS-CoV2 (Delta strain), a hybrid sequence of SARS-CoV-1 and SARS-CoV-2 (Delta strain) was designed. Spike protein variants were produced by inserting each of the designed hybrid sequences (Designs 1 to 5) into the RBD of the SARS-CoV-2 Delta strain spike protein (ectodomain, i.e., a construct lacking the transmembrane domain and the cytoplasmic tail), and neutralizing activity assay against SARS-CoV-1 and SARS-CoV2 was performed.

Using Expi293F cells, the spike proteins listed in the table below were prepared according to the method described in Example 1. It should be noted that amino acid residues at positions 1 to 13 of each full-length amino acid sequence are signal sequences, and are not included in the prepared antigen.

**[Table 19]**

| Name | RBD Sequence | SEQ ID NO. of Full Length Sequence |
|---|---|---|
| H-00120 | Delta Strain (SEQ ID NO: 28) | SEQ ID NO: 33 |
| H-00145 | Design 1 (SEQ ID NO: 29) | SEQ ID NO: 34 |
| H-00146 | Design 2 (SEQ ID NO: 30) | SEQ ID NO: 35 |
| H-00147 | Design 3 (SEQ ID NO: 31) | SEQ ID NO: 36 |
| H-00148 | Design 4 (SEQ ID NO: 32) | SEQ ID NO: 37 |
| H-00149 | Design 5 (SEQ ID NO: 3) | SEQ ID NO: 38 |

The spike proteins which were produced above were immunized into mice in the method as described in Example 4 and the neutralizing antibody titers in the serum were measured. Each administration group is as in Groups 1 to 6. It should be noted that Groups 5 and 6 are the coronavirus spike protein variants according to the present invention, Groups 2 to 4 are Reference Examples, and Group 1 is a Comparative Example.
(Group 1) H-00120,
(Group 2) H-00145,
(Group 3) H-00146,
(Group 4) H-00147,
(Group 5) H-00148,
(Group 6) H-00149.

The results showed that H-00148 (Group 5) and H-00149 (Group 6) induced neutralizing antibodies not only against wild-type D614G but also against SARS-CoV-1 (Fig. 1). In particular, it was found that the antigen H-00149 of Group 6 had high neutralizing antibody-inducing activity against the pseudoviruses of each strain tested.

### [Example 6]

### Neutralizing Antibody Induction Assay Using RBD Chimera

To check whether the RBDs of Design 5 and Design 4 alone can induce neutralizing antibodies against both SARS-CoV-1 and SARS-CoV-2, a neutralizing activity assay was performed using antigens consisting only of the RBD. RBD sequences for the RBDs of Designs 1, 2, 4, and 5 were produced, and, as controls, the RBD sequences of the Delta strain and SARS-CoV-1 were also produced. Specifically, each RBD listed in the table below was prepared according to the method of Example 1, and nanoparticle formation was performed according to the method of Example 2.

**[Table 20]**

| Name | RBD Sequence | Full-Length Sequence |
|---|---|---|
| H-00033 | Delta strain (sequence lacking three N-terminal amino acids of SEQ ID NO: 28) | SEQ ID NO: 40 |
| H-00082 | SARS-CoV-1 Strain (SEQ ID NO: 58) | SEQ ID NO: 41 |
| H-00078 | Design 1 (SEQ ID NO: 29) | SEQ ID NO: 42 |
| H-00079 | Design 2 (SEQ ID NO: 30) | SEQ ID NO: 43 |
| H-00081 | Design 4 (SEQ ID NO: 32) | SEQ ID NO: 44 |
| H-00428 | Design 5 (SEQ ID NO: 3) | SEQ ID NO: 45 |

The spike proteins which were produced above were immunized into mice in the method as described in Example 4 and the neutralizing antibody titers in the serum were measured. Each administration group is as in Groups 1 to 8. It should be noted that Groups 6 and 7 are the coronavirus spike protein variants according to the present invention, Groups 4 and 5 are Reference Examples, and Groups 1 to 3 and 8 are Comparative Examples.
(Group 1) RBD antigen from SARS-CoV-2 Delta strain (H-00033),
(Group 2) RBD antigen of SARS-CoV-1 (H-00082, SARS-1 described in the drawing),
(Group 3) Antigen of equal mixture of SARS-CoV-2 Delta strain RBD antigen (H-00033) and SARS-CoV-1 antigen (H-00082),
(Group 4) H-00078,
(Group 5) H-00079,
(Group 6) H-00081,
(Group 7) H-00428,
(Group 8) Ancestral strain S-full antigen (SEQ ID NO: 64, prepared according to the method described in Example 3)

The results showed that among the monovalent vaccines, H-00079 (Design 2), H-00081 (Design 4), and H-00428 (Design 5) induced neutralizing antibodies not only against wild-type D614G but also against SARS-CoV-1, with H-00428 (Design 5) in particular inducing balanced neutralizing antibodies against both SARS-CoV-1 and SARS-CoV-2 (Fig. 2). Thus, it was found that the RBDs of Design 5 and Design 4 are excellent antigen sequences having neutralizing antibody-inducing activity against both SARS-CoV-1 and SARS-CoV-2, regardless of whether they are administered as full-length spike proteins or as RBD-only antigens.

### [Example 7]

### Effect 1 of NTD Substitution on Neutralizing Antibody-Inducing Activity

To investigate the effect of the spike protein NTD on the induction of neutralizing activity, the antigens listed in the table below, in which the NTD of the Delta strain spike protein (ectodomain) was replaced, were prepared according to Example 1. It should be noted that amino acid residues at positions 1 to 13 of each amino acid sequence are signal sequences, and are not included in the prepared antigen.

**[Table 21]**

| Name | NTD | Full-Length Sequence |
|---|---|---|
| H-00120 | Derived from Delta Strain | SEQ ID NO: 33 |
| H-00545 | Derived from BA.5 Strain | SEQ ID NO: 46 |
| H-00632 | Derived from SARS-CoV-1 | SEQ ID NO: 47 |

The spike proteins which were produced above were immunized into mice in the method as described in Example 4 and the neutralizing antibody titers in the serum were measured. Each administration group is as in Groups 1 to 3. It should be noted that Groups 2 and 3 are Reference Examples used to examine the effect of a NTD mutation on neutralizing antibody-inducing activity, and Group 1 is a Comparative Example.
(Group 1) Spike ectodomain of SARS-CoV-2 Delta strain (H-00120),
(Group 2) Ectodomain (H-00545, SEQ ID NO: 46) in which the NTD of the SARS-CoV-2 Delta strain spike protein is replaced with the NTD sequence of the BA.5 strain,
(Group 3) Ectodomain (H-00632, SEQ ID NO: 47) in which the NTD sequence of the SARS-CoV-2 Delta strain spike protein has been replaced with the NTD sequence of SARS-CoV-1.

The results showed that, compared with H-00120, H-00545 exhibited an increased neutralizing antibody titer against BA.5, whereas H-00632 exhibited an increased neutralizing antibody titer against SARS-CoV-1 (Fig. 3). This result indicates that NTD of the spike protein contributes to neutralizing antibody-inducing activity.

### [Example 8]

### Effect 2 of NTD Substitution on Neutralizing-Antibody Inducing Activity

To investigate the effect of replacing the spike protein NTD on the neutralizing activity specificity of the RBD, the antigens listed in the table below were prepared according to Example 1. It should be noted that amino acid residues at positions 1 to 13 of each amino acid sequence are signal sequences, and are not included in the prepared antigen.

**[Table 22]**

| Name | NTD | RBD | SD1/2-S2 | Full-Length Sequence |
|---|---|---|---|---|
| H-00120 | Delta Strain | Delta Strain | Delta Strain | SEQ ID NO: 33 |
| H-00445 | BA.5 Strain | Delta Strain | BA.5 Strain | SEQ ID NO: 48 |
| H-00557 | BA.5 Strain | Design 5 | BA.5 Strain | SEQ ID NO: 49 |
| H-00556 | BA.5 Strain | Design 4 | BA.5 Strain | SEQ ID NO: 50 |

The spike proteins which were produced above were immunized into mice in the method as described in Example 4 and the neutralizing antibody titers in the serum were measured. Each administration group is as in Groups 1 to 5 below. It should be noted that Groups 3 and 4 are the coronavirus spike protein variants according to the present invention, Group 2 is a Reference Example, and Groups 1 and 5 are Comparative Examples.
(Group 1) H-00120,
(Group 2) Ectodomain (H-00445) in which the NTD and S2 of the SARS-CoV-2 Delta strain spike protein are replaced with the sequences of the BA.5 strain,
(Group 3) Ectodomain (H-00557) in which the NTD and SD1/2-S2 of H-00149 have been replaced with the sequence of the BA.5 strain,
(Group 4) Ectodomain in which NTD of H-00148 and SD1/2-S2 were replaced by the sequence of BA.5 strain (H-00556),
(Group 5) Ancestral strain S-full antigen prepared by BEVS.

The results showed that H-00557, which has Design 5 in the RBD, exhibited neutralizing antibody-inducing activity not only against D614G but also against both BA.4/5 and SARS-CoV-1, inducing balanced neutralizing activity against all virus strains (Fig. 4). Based on the above results, it was shown that the sequence introduced into the NTD not only induces neutralizing antibodies against the corresponding strain, but also, when combined with the chimeric RBD sequence identified in Examples 5 and 6, simultaneously induces neutralizing antibodies against both SARS-CoV-1 and SARS-CoV-2.

### [Example 9]

### Effect 1 of Introducing Amino Acid Mutations on Neutralizing Antibody-Inducing Activity

In order to investigate the effect of deletion of amino acid residues at glycosylation sites of the spike protein on neutralizing activity specificity, the antigens shown in the table below were prepared according to Example 1. It should be noted that amino acid residues at positions 1 to 13 of each amino acid sequence are signal sequences, and are not included in the prepared antigen.

**[Table 23]**

| Name | Position 167 | Position 331 | Full-Length Sequence |
|---|---|---|---|
| H-00120 | T | N | SEQ ID NO: 33 |
| H-00161 | L | N | SEQ ID NO: 51 |
| H-00168 | T | Q | SEQ ID NO: 52 |

In the above table, positions 167 and 331 refer to the amino acid residues corresponding to positions 167 and 331 in the amino acid sequence of the ancestral strain spike protein. The spike proteins which were produced above were immunized into mice in the method as described in Example 4 and the neutralizing antibody titers in the serum were measured. Each administration group is as in Groups 1 to 3. It should be noted that Groups 2 and 3 are Reference Examples used to examine the effect of amino acid mutations at positions 167 and 331, and Group 1 is a Comparative Example.
(Group 1) Spike ectodomain of SARS-CoV-2 Delta strain (H-00120),
(Group 2) Spike ectodomain (H-00161) in which a T167L mutation has been introduced into the SARS-CoV-2 Delta strain, or
(Group 3) Spike ectodomain in which an N331Q mutation has been introduced into the SARS-CoV-2 Delta strain.

The results showed that H-00161 and H-00168 have higher neutralizing antibody-inducing activity against BA.4/5 than H-00120 (Fig. 5).

### [Example 10]

### Effect 2 of Introducing Amino Acid Mutations on Neutralizing Antibody-Inducing Activity

In order to investigate the effect of deletion of amino acid residues at glycosylation sites of the spike protein on neutralizing activity specificity, the antigens shown in the table below were prepared according to Example 1. It should be noted that amino acid residues at positions 1 to 13 of each amino acid sequence are signal sequences, and are not included in the prepared antigen.

**[Table 24]**

| Name | Position 1173 | Full-Length Sequence |
|---|---|---|
| H-00120 | N | SEQ ID NO: 33 |
| H-00189 | L | SEQ ID NO: 53 |

In the above table, position 1173 refers to the amino acid residue corresponding to position 1173 in the amino acid sequence of the ancestral strain spike protein. The spike proteins which were produced above were immunized into mice in the method as described in Example 4 and the neutralizing antibody titers in the serum were measured. Each administration group is as listed in Groups 1 and 2. It should be noted that Group 2 is a Reference Example used to examine the effect of a N1173L mutation, and Group 1 is a Comparative Example.
(Group 1) Spike ectodomain of SARS-CoV-2 Delta strain (H-00120),
(Group 2) Spike ectodomain (H-00189) in which a N1173L mutation has been introduced into SARS-CoV-2 Delta strain.

The results showed that H-00189 has higher neutralizing antibody-inducing activity against BA.4/5 than H-00120 (Fig. 6).

### [Example 11]

### Effect 3 of Introducing Amino Acid Mutations on Neutralizing Antibody Induction

In order to investigate the effect of deletion of amino acid residues at glycosylation sites of the spike protein on neutralizing activity specificity, the antigens shown in the table below were prepared according to the method described in Example 1. It should be noted that amino acid residues at positions 1 to 13 of each amino acid sequence are signal sequences, and are not included in the prepared antigen.

**[Table 25]**

| Name | Position 1074 | Position 1098 | Full-Length Sequence |
|---|---|---|---|
| H-00120 | N | N | SEQ ID NO: 33 |
| H-00511 | Q | T | SEQ ID NO: 54 |

In the above table, positions 1074 and 1098 refer to the amino acid residues corresponding to positions 1074 and 1098 in the amino acid sequence of the ancestral strain spike protein. The spike proteins which were produced above were immunized into mice in the method as described in Example 4 and the neutralizing antibody titers in the serum were measured. Each administration group is as listed in Groups 1 and 2. It should be noted that Group 2 is a Reference Example used to examine the effect of a N1074Q mutation and a N1074T mutation, and Group 1 is a Comparative Example.
(Group 1) Spike ectodomain of SARS-CoV-2 Delta strain (H-00120),
(Group 2) Spike ectodomain (H-00511) in which a N1074Q mutation and a N1074T mutation are introduced into the SARS-CoV-2 Delta strain.

The results showed that H-00511 has higher neutralizing antibody-inducing activity against BA.4/5 than H-00120 (Fig. 7). The above results demonstrate that neutralizing antibody-inducing activity can be improved by amino acid substitutions that eliminate glycosylation sites.

### [Example 12]

### Effect 1 of Structure-Stabilizing Mutations on Neutralizing-Antibody Inducing Activity

In order to investigate the effect of the following amino acid residue substitutions, which may contribute to structural stabilization of the spike protein, on neutralizing activity specificity, the antigens in the table below were made according to Example 1. It should be noted that amino acid residues at positions 1 to 13 of each amino acid sequence are signal sequences, and are not included in the prepared antigen.

**[Table 26]**

| Name | Position 795 | Position 965 | Position 1003 | Full-Length Sequence |
|---|---|---|---|---|
| H-00120 | K | Q | S | SEQ ID NO: 33 |
| H-00346 | K | C | C | SEQ ID NO: 55 |
| H-00399 | P | Q | Q | SEQ ID NO: 56 |

In the above table, positions 795, 965, and 1003 refer to the amino acid residues corresponding to positions 795, 965, and 1003 in the amino acid sequence of the ancestral strain spike protein. The spike proteins which were produced above were immunized into mice in the method as described in Example 4 and the neutralizing antibody titers in the serum were measured. Each administration group is as in Groups 1 to 3. It should be noted that Groups 2 and 3 are Reference Examples used to examine the effect of structure-stabilizing mutations, and Group 1 is a Comparative Example.
(Group 1) Spike ectodomain of SARS-CoV-2 Delta strain (H-00120),
(Group 2) Spike ectodomain (H-00346) in which a Q965C mutation and a S1003C mutation have been introduced into the SARS-CoV-2 Delta strain,
(Group 3) Spike ectodomain (H-00399) in which a K795P mutation has been introduced into the SARS-CoV-2 Delta strain.

The results showed that H-00346 and H-00399 have higher neutralizing antibody-inducing activity against BA.4/5 than H-00120 (Fig. 8).

### [Example 13]

### Effect 2 of Structure-Stabilizing Mutations on Neutralizing-Antibody Inducing Activity

In order to investigate the effect of the following amino acid residue substitutions, which may contribute to structural stabilization of the spike protein, on neutralizing activity specificity, the antigens in the table below were made according to Example 1. It should be noted that amino acid residues at positions 1 to 13 of each amino acid sequence are signal sequences, and are not included in the prepared antigen.

**[Table 27]**

| Name | Position 732 | Full-Length Sequence |
|---|---|---|
| H-00120 | T | SEQ ID NO: 33 |
| H-00391 | P | SEQ ID NO: 57 |

In the above table, position 732 refers to the amino acid residue corresponding to position 732 in the amino acid sequence of the ancestral strain spike protein. The spike proteins which were produced above were immunized into mice in the method as described in Example 4 and the neutralizing antibody titers in the serum were measured. Each administration group is as in Groups 1 to 3. It should be noted that Group 2 is a Reference Example used to examine the effect of structure-stabilizing mutations, and Group 1 is a Comparative Example.
(Group 1) Spike ectodomain of SARS-CoV-2 Delta strain (H-00120),
(Group 2) Spike ectodomain (H-00391) in which a T732P mutation has been introduced into SARS-CoV-2 Delta strain.

The results showed that H-00391 has higher neutralizing antibody-inducing activity against BA.4/5 than H-00120 (Fig. 9). The above results indicate that neutralizing antibody-inducing activity can be improved by substituting each of the above-mentioned amino acid residues in the spike protein.

### [Example 14]

### Neutralizing Antibody Induction Assay 1 of Antigens Designed by Combining the Above Results

Based on the findings in Examples 5 to 13, the spike protein variants described in the table below were designed and antigen proteins were produced according to Example 1. It should be noted that amino acid residues at positions 1 to 13 of each amino acid sequence are signal sequences, and are not included in the prepared antigen.

**[Table 28]**

| Name | Full-Length Sequence |
|---|---|
| H-00646 | SEQ ID NO: 21 |
| H-00657 | SEQ ID NO: 22 |
| H-00669 | SEQ ID NO: 23 |
| H-00672 | SEQ ID NO: 24 |
| H-00675 | SEQ ID NO: 25 |

The spike proteins which were produced above or each antigen prepared by the method described in Example 3 were immunized into mice in the method as described in Example 4 and the neutralizing antibody titers in the serum were measured. Each administration group is as in Groups 1 to 7 below. It should be noted that Groups 3 to 7 are the coronavirus spike protein variants according to the present invention, and Groups 1 and 2 are Comparative Examples.
(Group 1) Ancestral strain S-full antigen (SEQ ID NO: 64, prepared according to the method described in Example 3),
(Group 2) Bivalent vaccine obtained by mixing equal amounts of the ancestral strain S-full antigen (the same as Group 1) and the BA.5 strain S-full antigen (SEQ ID NO: 63, prepared according to the method described in Example 3),
(Group 3) H-00646,
(Group 4) H-00657,
(Group 5) H-00669,
(Group 6) H-00672,
(Group 7) H-00675.

The results showed that the ancestral strain vaccine in Group 1 induced neutralizing antibodies only against a limited set of strains, and the bivalent vaccine in Group 2 did not induce sufficient neutralizing activity against the various SARS-CoV-2 strains or other sarbecoviruses (SARS-CoV-1, WIV-1). Meanwhile, H-00646, H-00657, H-00669, H-00672, and H-00675 exhibited high neutralizing antibody-inducing activity against sarbecoviruses in general (Fig. 10).

### [Example 15]

### Neutralizing Antibody Induction Assay 2 of Antigens Designed by Combining the Above Results

Based on the findings in Examples 5 to 13, the spike protein variants described in the table below were designed and antigen proteins were produced according to Example 1. It should be noted that amino acid residues at positions 1 to 13 of each amino acid sequence are signal sequences, and are not included in the prepared antigen.

**[Table 29]**

| Name | Full-Length Sequence |
|---|---|
| H-00739 | SEQ ID NO: 26 |
| H-00740 | SEQ ID NO: 27 |

The spike proteins which were produced above or each antigen prepared by the method described in Example 3 were immunized into mice in the method as described in Example 4 and the neutralizing antibody titers in the serum were measured. Each administration group is as in Groups 1 to 3. It should be noted that Groups 2 and 3 are the coronavirus spike protein variants according to the present invention, and Groups 1 is a Comparative Example.
(Group 1) Ancestral strain S-full antigen (SEQ ID NO: 64, prepared according to the method described in Example 3),
(Group 2) H-00739,
(Group 3) H-00740.

The results showed that while the ancestral strain vaccine induced neutralizing antibodies only against a limited set of strains, H-00739 and H-00740 exhibited high neutralizing antibody-inducing activity against all sarbecoviruses (Fig. 11).

### [Example 16]

### Neutralizing Antibody Induction Assay of Antigens Administered In Vivo as mRNA

The following experiment was conducted to evaluate the neutralizing antibody-inducing activity of the above-designed antigens when administered in vivo as mRNA.

### (1) Preparation of Template DNA for In Vitro Transcription (IVT)

To produce the template DNA for in vitro transcription (IVT), the plasmid DNA was amplified by PCR and then purified. A DNA fragment including a sequence in which a T7 promoter sequence, a 5'-UTR sequence, a KOZAK sequence, an ORF, and a 3'-UTR sequence are sequentially linked was introduced into the plasmid. To a solution of 1.8 ng of the plasmid dissolved in Nuclease-free water (207 µL), added were 2× Prime STAR MAX DNA polymerase Premix (120 µL, Takara catalog #R045), a 10 µM sense primer (9 µL, SEQ ID NO: 60), and a 10 µM antisense primer (9 µL, SEQ ID NO: 61). After incubation at 98°C for 2 minutes, PCR was performed for 35 cycles consisting of 98°C for 10 seconds, 55°C for 5 seconds, and 72°C for 10 seconds, thereby amplifying DNA encoding the S full of H-00672 (SEQ ID NO: 62, a sequence in which the C-terminal foldon sequence and His tag sequence of H-00672 are deleted and a transmembrane domain and a cytoplasmic tail are added). After completion of the reaction, the template DNA was purified using NucleoSpin (registered trademark) Gel and PCR Clean-up (Takara catalog #740609.250).

### (2) Preparation of SARS-CoV-2S-full mRNA-001 (SEQ ID NO: 59) by In Vitro Transcription

The 358 µg/mL template DNA obtained above (8.4 µL), 75 mM ATP (30 µL, Thermo Fisher catalog #AMB13345), 75 mM GTP (30 µL, Thermo Fisher catalog #AMB13345), 75 mM CTP (30 µL, Thermo Fisher catalog #AMB13345), 100 mM N1-methylpseudouridine triphosphate (22.5 µL), Nuclease-free water (119 µL), 10X Reaction Buffer (30 µL, Thermo Fisher catalog #AMB13345), and Enzyme mix T7 RNA Polymerase (30 µL, Thermo Fisher catalog #AMB13345) were mixed and incubated at 37°C for 2 hours. TURBO (registered trademark) DNase (15 µL, Thermo Fisher catalog #AM2239) was added, and the mixture was incubated at 37°C for 15 minutes. A 7.5 M LiCl solution (150 µL, Thermo Fisher catalog #AM9480) was added, and the mixture was left to stand overnight at -20°C. After centrifugation (4°C, 15,000 × g, 15 minutes), the supernatant was discarded, 70% ethanol was added, and after centrifugation (4°C, 15,000 × g, 10 minutes), the supernatant was discarded and air-dried. After the obtained residue was dissolved in Nuclease-free water, a portion of the resulting solution (285 µL, corresponding to 450 µg as calculated by UV measurement), Nuclease-free water (254 µL), 10 mM GTP (90 µL, New England Biolabs catalog #M2080), 20 mM SAM (90 µL, New England Biolabs catalog #M2080), 10X Capping Buffer (90 µL, New England Biolabs catalog #M2080), Vaccinia Capping Enzyme (10 U/µL, 45 µL, New England Biolabs catalog #M2080), and mRNA Cap2'-O-Methyltransferase (50 U/µL, 45 µL, New England Biolabs catalog #M0366) were mixed and incubated at 37°C for 2 hours. A 7.5 M LiCl solution (450 µL, Thermo Fisher catalog #AM9480) was added, and the mixture was left to stand overnight at -20°C. After centrifugation (4°C, 15,000 × g, 15 minutes), the supernatant was discarded, 70% ethanol was added, and after centrifugation (4°C, 15,000 × g, 10 minutes), the supernatant was discarded and air-dried. After the obtained residue was dissolved in Nuclease-free water, the resulting solution (450 µL, corresponding to 421 µg as calculated by UV measurement), 10X Antarctic Phosphatase Reaction Buffer (51 µL, New England Biolabs catalog #B0289), and Antarctic Phosphatase (10 µL, New England Biolabs catalog #M0289) were mixed and incubated at 37°C for 30 minutes. Thereafter, the reaction mixture was processed using the Monarch (registered trademark) RNA Cleanup Kit (New England Biolabs catalog #T2050) according to the manufacturer's manual, thereby obtaining the target mRNA-001. The mRNA obtained was analyzed by TapeStation (Agilent) to confirm the desired length.

### (3) Preparation of mRNA Encapsulated Nucleic Acid Lipid Particles With SARS-CoV-2 S Full mRNA

SM-102 (Medchemexpress, HY-134541) and a cationic lipid described in WO 2022/168884 (hereinafter referred to as "lipid"), distearoylphosphatidylcholine (1,2-Distearoyl-sn-glycero-3-phosphocholine, hereinafter referred to as DSPC, NOF CORPORATION), Cholesterol (hereinafter referred to as "Chol"; NIPPON FINE CHEMICAL CO., LTD.), and 1,2-Dimyristoyl-sn-Glycero-3-Methoxypolyethylene Glycol having a polyethylene glycol molecular weight of approximately 2000 (hereinafter referred to as "PEG-DMG"; NOF CORPORATION) were dissolved in ethanol at a molar ratio of DSPC:Chol:Lipid:PEG-DMG = 7.9:30.2:60.4:1.5 so as to give a total lipid concentration of 13 mM.

Meanwhile, the mRNA obtained by the above-described preparation of mRNA-001 was adjusted to a concentration of 35 µg/mL using an acetate buffer (pH 4.0).

The above lipid solution and mRNA solution were mixed in a microfluidic channel using NanoAssemblr (registered trademark) IGNITE^{™} (Precision Nanosystems Inc.) at a volume ratio of 1:3, thereby obtaining a crude dispersion of nucleic acid lipid particles. The dispersion of nucleic acid lipid particles was dialyzed against approximately 300 volumes of 4 mM phosphate-buffered saline (pH 7.4) for 12 to 18 hours using a Slide-A-Lyzer^{™} Dialysis Cassette G2 (MWCO: 10 kD; Spectra/Por) to remove ethanol, thereby obtaining a purified dispersion of mRNA-encapsulated nucleic acid lipid particles, which was subjected to the following neutralizing antibody induction experiments.

### (4) Neutralizing Antibody Induction Assay Using mRNA

The mRNA produced by the method, or the H-00672 antigen produced by the method described in Example 1, or the ancestral strain antigen prepared by the method described in Example 3, was administered to mice for immunization according to the method described in Example 4, and the serum neutralizing antibody titers were measured. Each administration group is as in Groups 1 to 4.
(Group 1) Ancestral strain S-full antigen (SEQ ID NO: 64, prepared according to the method described in Example 3),
(Group 2) H-00672 protein antigen (SEQ ID NO: 24, prepared according to the method described in Example 1, wherein amino acid residues at positions 1 to 13 of the amino acid sequence constitute a signal sequence and are not included in the prepared antigen.),
(Group 3) 0.3 µg of mRNA antigen (nucleic acid sequence: SEQ ID NO: 59, amino acid sequence: SEQ ID NO: 62, dispersion prepared above)
(Group 4) 3 µg of mRNA antigen (nucleic acid sequence: SEQ ID NO: 59, amino acid sequence: SEQ ID NO: 62, dispersion prepared as described above).

It should be noted that Group 2 is the coronavirus spike protein variant according to the present invention, Groups 3 and 4 are nucleic acids encoding the coronavirus spike protein variant according to the present invention, and Group 1 is a Comparative Example.

In this assay, for (Group 3) and (Group 4), a booster immunization was performed 3 weeks after the primary immunization, and serum was collected 2 weeks thereafter to measure neutralizing antibody titers.

The results showed that both the protein antigens and the mRNA antigens induced high neutralizing antibody-inducing activity against all evaluated sarbecoviruses (Fig. 12).

### [Example 17]

### Comparison of Neutralizing Antibody-Inducing Activity of Antigens Expressed in Expi293F Cells and Antigens Prepared by BEVS

For H-00672, the neutralizing antibody-inducing activity of the antigen expressed in Expi293F cells as described in Example 1 was compared with that of the antigen prepared using BEVS as described in Example 3. Mice were immunized according to the method described in Example 4, and serum neutralizing antibody titers were measured. Each administration group is as in Groups 1 to 3. It should be noted that Groups 2 and 3 are the coronavirus spike protein variants according to the present invention, and Group 1 is a Comparative Example.
(Group 1) Ancestral strain S-full antigen (SEQ ID NO: 64, prepared according to the method described in Example 3),
(Group 2) H-00672 (SEQ ID NO: 24, prepared according to the method described in Example 1, wherein amino acid residues at positions 1 to 13 of the amino acid sequence constitute a signal sequence and are not included in the prepared antigen.),
(Group 3) H-00672 (SEQ ID NO: 24, prepared according to the method described in Example 3, wherein amino acid residues at positions 1 to 13 of the amino acid sequence constitute a signal sequence and are not included in the prepared antigen.).

The results showed that antigens produced by all manufacturing methods exhibited high neutralizing antibody-inducing activity against the evaluated pseudoviruses (Fig. 13).

### [Example 18]

### Consideration of Adjuvant

To evaluate adjuvants, using the H-00672 antigen, A-910823 (Group 1 below), which was described in Example 4 and used for non-mRNA immunizations in Examples 5 to 17 above, and the following two alternative adjuvants (Groups 2 and 3 below) were compared. Except for the type of adjuvant, mice were immunized according to the method described in Example 4, and serum neutralizing antibody titers were measured. Each administration group is as in Groups 1 to 3.
(Group 1) Squalene-based emulsion adjuvant A-910823,
(Group 2) Adjuvant obtained by mixing an aluminum salt, Alum, with an oligodeoxynucleotide, ODN2006,
(Group 3) Adjuvant of mixture of aluminum salt Alum and oligodeoxynucleotide CpG1018.

Note that each of Groups 1 to 3 is an immunogenic composition containing the coronavirus spike protein variant according to the present invention.

The results showed that the neutralizing antibody-inducing activity against the pseudovirus was similar for all adjuvants tested (Fig. 14).

### [Example 19]

Based on the findings obtained in Examples 5 to 15, a total of 58 coronavirus spike protein variants according to the present invention as described in the table below were designed and each spike protein was prepared according to Example 1. It should be noted that amino acid residues at positions 1 to 13 of each amino acid sequence are signal sequences, and are not included in the prepared antigen.

**[Table 30]**

| Name | SEQ ID NO. | Name | SEQ ID NO. |
|---|---|---|---|
| H-00885 | 65 | H-00987 | 94 |
| H-00954 | 66 | H-00980 | 95 |
| H-00901 | 67 | H-00895 | 96 |
| H-00881 | 68 | H-00870 | 97 |
| H-01059 | 69 | H-00967 | 98 |
| H-00952 | 70 | H-00905 | 99 |
| H-00986 | 71 | H-00886 | 100 |
| H-00945 | 72 | H-00951 | 101 |
| H-01063 | 73 | H-00890 | 102 |
| H-00854 | 74 | H-00920 | 103 |
| H-00953 | 75 | H-00862 | 104 |
| H-00874 | 76 | H-00893 | 105 |
| H-00962 | 77 | H-00891 | 106 |
| H-00807 | 78 | H-00889 | 107 |
| H-00783 | 79 | H-00872 | 108 |
| H-00848 | 80 | H-00934 | 109 |
| H-00990 | 81 | H-00892 | 110 |
| H-00916 | 82 | H-00956 | 111 |
| H-00794 | 83 | H-01017 | 112 |
| H-00914 | 84 | H-01024 | 113 |
| H-00958 | 85 | H-01030 | 114 |
| H-00909 | 86 | H-01036 | 115 |
| H-00917 | 87 | H-01039 | 116 |
| H-00955 | 88 | H-01044 | 117 |
| H-00915 | 89 | H-01046 | 118 |
| H-00997 | 90 | H-01047 | 119 |
| H-00789 | 91 | H-01048 | 120 |
| H-00829 | 92 | H-01049 | 121 |
| H-00976 | 93 | H-01112 | 122 |

The spike proteins which were produced above were immunized into mice in the method as described in Example 4 and the neutralizing antibody titers in the serum were measured. Note that the spike proteins of the viral strains used in the pseudovirus neutralization assay were the following three types: D614G, XBB.1, and SARS-1 shown in Table 18.

The results showed that all of the spike proteins listed in the above table exhibited high neutralizing antibody-inducing activity against the pseudoviruses of the three sarbecoviruses tested (Figs. 15 to 24).

### [Industrial Applicability]

The spike protein variant of the present invention, or an immunogenic composition containing the spike protein variant, exhibits an excellent effect in that it shows broad neutralizing antibody-inducing activity with a monovalent antigen.

### [Sequence Listing]

C:\Users\common\Documents\5591 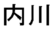 \678409\678409 Sequence Listing.xml

## Claims

1. A coronavirus spike protein variant comprising:
(a) a receptor binding domain (RBD) consisting of an amino acid sequence set forth in SEQ ID NO: 1;
(b) an RBD consisting of an amino acid sequence obtained by substituting, deleting, inserting, or adding one to several amino acids in the amino acid sequence set forth in SEQ ID NO: 1, and having neutralizing antibody-inducing activity against SARS-CoV-2 and SARS-CoV-1 equivalent to that of an RBD consisting of the amino acid sequence set forth in SEQ ID NO: 1; or
(c) an RBD consisting of an amino acid sequence that has at least 90% sequence identity to the amino acid sequence set forth in SEQ ID NO: 1, and having neutralizing antibody-inducing activity against SARS-CoV-2 and SARS-CoV-1 equivalent to that of the RBD consisting of the amino acid sequence set forth in SEQ ID NO: 1.

2. The spike protein variant according to claim 1, wherein the amino acid sequence set forth in SEQ ID NO: 1 is an amino acid sequence set forth in any one of SEQ ID NOs: 2 to 5.

3. The spike protein variant according to claim 1 or 2,comprising an amino acid sequence having at least 90% sequence identity to an amino acid sequence consisting of amino acid residues at positions 14 to 1211 of the amino acid sequence set forth in SEQ ID NO: 6.

4. The spike protein variant according to any one of claims 1 to 3, comprising an N-terminal domain (NTD) consisting of: (a) an amino acid sequence set forth in SEQ ID NO: 7;
(b) an amino acid sequence obtained by substituting, deleting, inserting, or adding one to several amino acids in the amino acid sequence set forth in SEQ ID NO: 7; or
(c) an amino acid sequence having at least 90% sequence identity to the amino acid sequence set forth in SEQ ID NO: 7.

5. The spike protein variant according to claim 4, wherein the amino acid sequence set forth in SEQ ID NO: 7 is an amino acid sequence set forth in either SEQ ID NO: 8 or 9.

6. The spike protein variant according to any one of claims 1 to 5, comprising a region of SD1, SD2, and S2 subunits consisting of: (a) an amino acid sequence set forth in SEQ ID NO: 10;
(b) an amino acid sequence obtained by substituting, deleting, inserting, or adding one to several amino acids in the amino acid sequence set forth in SEQ ID NO: 10; or
(c) an amino acid sequence having at least 90% sequence identity to the amino acid sequence set forth in SEQ ID NO: 10.

7. The spike protein variant according to claim 6, wherein the amino acid sequence set forth in SEQ ID NO: 10 is an amino acid sequence set forth in any one of SEQ ID NOs: 11 to 15.

8. The spike protein variant according to any one of claims 1 to 5, comprising a region of SD1, SD2, and S2 subunits consisting of: (a) an amino acid sequence set forth in SEQ ID NO: 16;
(b) an amino acid sequence obtained by substituting, deleting, inserting, or adding one to several amino acids in the amino acid sequence set forth in SEQ ID NO: 16; or
(c) an amino acid sequence having at least 90% sequence identity to the amino acid sequence set forth in SEQ ID NO: 16.

9. The spike protein variant according to claim 8, having amino acid substitutions corresponding to Q434C substitution and S472C substitution in the amino acid sequence set forth in SEQ ID NO: 16.

10. The spike protein variant according to claim 8 or 9, having amino acid substitution corresponding to K455P substitution and/or V456P substitution in the amino acid sequence set forth in SEQ ID NO: 16.

11. The spike protein variant according to any one of claims 8 to 10, having one or more amino acid substitutions corresponding to substitutions selected from the group consisting of F286P substitution, A361P substitution, A368P substitution, and A411P substitution in the amino acid sequence set forth in SEQ ID NO: 16.

12. The spike protein variant according to any one of claims 8 to 11, having amino acid substitution corresponding to T201P substitution and/or K264P substitution in the amino acid sequence set forth in SEQ ID NO: 16.

13. The spike protein variant according to any one of claims 8 to 12, wherein one or more amino acid residues corresponding to RRAR (SEQ ID NO: 17) at positions 151 to 154 of the amino acid sequence set forth in SEQ ID NO: 16 have amino acid mutations and are not cleaved by a furin protease.

14. The spike protein variant according to claim 13, wherein amino acid residues corresponding to RRAR at positions 151 to 154 of the amino acid sequence set forth in SEQ ID NO: 16 are substituted with GSAS (SEQ ID NO: 18).

15. The spike protein variant according to any one of claims 8 to 14, having amino acid substitutions corresponding to one or more amino acid substitutions selected from the group consisting of N543Q substitution, N567Q substitution, and N642L substitution in the amino acid sequence set forth in SEQ ID NO: 16.

16. The spike protein variant according to any one of claims 1 to 15, wherein a transmembrane domain and a cytoplasmic tail are deleted.

17. The spike protein variant according to claim 16, wherein a trimerization domain is added at the C-terminus.

18. The spike protein variant according to any one of claims 1 to 17, wherein a His tag is added at the C-terminus.

19. The spike protein variant according to any one of claims 1 to 18, comprising amino acid residues at positions 14 to 1236 of an amino acid sequence set forth in any one of SEQ ID NOs: 21 to 27.

20. A coronavirus spike protein variant having at least 99% sequence identity to amino acid residues at positions 14 to 1236 of an amino acid sequence set forth in SEQ ID NO: 26, and comprising amino acid sequences of amino acid residues at positions 14 to 324 and positions 528 to 1236 of an amino acid sequence set forth in SEQ ID NO: 26.

21. The spike protein variant according to claim 20, comprising amino acid residues at positions 14 to 1236 of an amino acid sequence set forth in any one of SEQ ID NOs: 65, 68, 71, 74, 76, 82, 84, 87, 89, 92, 93, 94, 96, 97, 100, 102 to 104, or 106 to 110.

22. A coronavirus spike protein variant having at least 99% sequence identity to amino acid residues at positions 14 to 1236 of an amino acid sequence set forth in SEQ ID NO: 26, and comprising amino acid residues at positions 325 to 527 of an amino acid sequence set forth in SEQ ID NO: 26.

23. The spike protein variant according to claim 22, comprising amino acid residues at positions 14 to 1236 of the amino acid sequence set forth in any one of SEQ ID NOs: 66, 67, 69, 70, 72, 73, 75, 77 to 81, 83, 85, 86, 88, 90, 91, 95, 98, 99, 101, 105, and 111 to 122.

24. An immunogenic composition comprising the spike protein variant according to any one of claims 1 to 23.

25. The immunogenic composition according to claim 24, comprising a single type of spike protein variant according to claims 1 to 23 as an antigen derived from a coronavirus spike protein, and not comprising any other antigen derived from the coronavirus spike protein.

26. The immunogenic composition according to claim 24 or 25, further comprising an adjuvant.

27. The immunogenic composition according to claim 26, wherein the adjuvant is an adjuvant comprising squalene, tocopherol, and polysorbate 80.

28. A nucleic acid encoding the spike protein variant according to any one of claims 1 to 23.

29. An expression vector comprising the nucleic acid according to claim 28.
